(19) Europäisches Patentamt / European Patent Office / Office européen des brevets

(11) EP 2 739 148 B1

(12) **EUROPÄISCHE PATENTSCHRIFT**

(45) Veröffentlichungstag und Bekanntmachung des Hinweises auf die Patenterteilung:
**28.09.2016 Patentblatt 2016/39**

(21) Anmeldenummer: **12741264.1**

(22) Anmeldetag: **31.07.2012**

(51) Int Cl.:
***A01N 43/90*** *(2006.01)* ***A01N 31/02*** *(2006.01)*
***A01P 1/00*** *(2006.01)* ***A61K 8/37*** *(2006.01)*
***A61Q 17/00*** *(2006.01)* ***A61Q 19/00*** *(2006.01)*
***C11D 3/20*** *(2006.01)*

(86) Internationale Anmeldenummer:
**PCT/EP2012/003252**

(87) Internationale Veröffentlichungsnummer:
**WO 2013/017263 (07.02.2013 Gazette 2013/06)**

(54) **ZUSAMMENSETZUNGEN ENTHALTEND ISOSORBIDMONOESTER UND ALIPHATISCHE VICINALE DIOLE**

COMPOSITIONS COMPRISING ISOSORBIDE MONOESTERS AND ALIPHATIC VICINAL DIOLS

COMPOSITIONS COMPRENANT DU MONOESTER D'ISOSORBIDE ET DES DIOLS ALIPHATIQUES VICINALS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.08.2011 DE 102011109493**

(43) Veröffentlichungstag der Anmeldung:
**11.06.2014 Patentblatt 2014/24**

(73) Patentinhaber: **CLARIANT INTERNATIONAL LTD**
**4132 Muttenz (CH)**

(72) Erfinder:
- **PILZ, Maurice Frederic**
  **60329 Frankfurt am Main (DE)**
- **KLUG, Peter**
  **63762 Großostheim (DE)**
- **SCHERL, Franz-Xaver**
  **84508 Burgkirchen (DE)**
- **GROHMANN, Joerg**
  **65527 Niedernhausen (DE)**

(74) Vertreter: **Holmes, Rosalind et al**
**Clariant Produkte (Deutschland) GmbH**
**Patent & License Management Chemicals**
**Industriepark Höchst, G 860**
**65926 Frankfurt am Main (DE)**

(56) Entgegenhaltungen:
**WO-A2-2010/108738 DE-A1-102009 022 445**

- **GIACOMETTI J ET AL: "Process for Preparing Nonionic Surfactant Sorbitan Fatty Acid Esters with and without Previous Sorbitol Cyclization", JOURNAL OF AGRICULTURAL AND FOOD CHEMISTRY, AMERICAN CHEMICAL SOCIETY, US, Bd. 44, 1. Januar 1996 (1996-01-01), Seiten 3950-3954, XP002278710, ISSN: 0021-8561, DOI: 10.1021/JF950314J**
- **Frieder W. Lichtenthaler: "Carbohydrates, Chapter 9: Carbohydrates as Organic Raw Materials" In: "Ullmann's Encyclopedia of Industrial Chemistry, Volume 6", 1. Januar 2003 (2003-01-01), Wiley-VCH, XP55040020, ISBN: 978-3-52-730385-4 Seiten 262-273, Seite 270, Spalte 2, Zeile 5 - Zeile 18**
- **PETER STOSS ET AL: "Regioselektive Acylierung von 1,4:3,6-Dianhydro-D-glucit", SYNTHESIS, Bd. 1987, Nr. 02, 1. Januar 1987 (1987-01-01), Seiten 174-176, XP55039551, ISSN: 0039-7881, DOI: 10.1055/s-1987-27878**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 739 148 B1

**Beschreibung**

Die vorliegende Erfindung betrifft Zusammensetzungen enthaltend Isosorbidmonoester und ein oder mehrere aliphatische vicinale Diole. Bei diesen Zusammensetzungen kann es sich beispielsweise um kosmetische, dermatologische oder pharmazeutische Zusammensetzungen handeln oder auch um Zusammensetzungen, die z. B. erst zur Herstellung von kosmetischen, dermatologischen oder pharmazeutischen Zusammensetzungen verwendet werden können. Weiterhin kann es sich auch um Pflanzenschutzformulierungen oder um Wasch- und Reinigungsmittel handeln. Die vorliegende Erfindung betrifft auch die Verwendung von Zusammensetzungen enthaltend Isosorbidmonoester und ein oder mehrere aliphatische vicinale Diole zum Konservieren von kosmetischen, dermatologischen oder pharmazeutischen Zusammensetzungen, Pflanzenschutzformulierungen oder Wasch- und Reinigungsmitteln.

In der Industrie werden Konservierungsmittel oder Biozide verwendet, um Produkte wie beispielsweise kosmetische, dermatologische oder pharmazeutische Zusammensetzungen, Pflanzenschutzformulierungen oder Wasch- und Reinigungsmittel gegen mikrobiellen Befall zu schützen. Es sind zahlreiche Konservierungsmittel für diesen Zweck bekannt. Beispielsweise ist bekannt, dass aliphatische vicinale Diole wie z. B. Ethylhexylglycerin hierfür verwendet werden können.

Nachteilig an der Verwendung vieler Konservierungsmittel ist jedoch, dass ihre Herstellung oftmals aufwändig ist und auf synthetischen Rohstoffen basiert. Zudem ist ihre konservierende Wirkung oftmals verbesserungsbedürftig, so dass hohe Einsatzkonzentrationen für eine ausreichende Konservierung notwendig sind.

Es bestand deshalb die Aufgabe, Zusammensetzungen zur Verfügung zu stellen, die eine vorteilhafte Konservierungsmittelleistung aufweisen oder eine vorteilhafte Stabilität gegen mikrobiellen Befall besitzen und sich zudem durch den Vorteil auszeichnen, dass sie zumindest teilweise auf nachwachsenden Rohstoffen basieren.

Überraschend wurde nun gefunden, dass diese Aufgabe gelöst wird durch Zusammensetzungen enthaltend

a) eine oder mehrere Verbindungen der Formel (I)

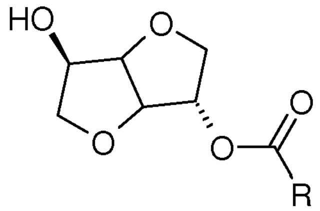

(I)

worin
der Rest R in Formel (I) ein linearer gesättigter Alkylrest mit 7 bis 9, bevorzugt 7 Kohlenstoffatomen ist, und

b) ein oder mehrere aliphatische vicinale Diole ausgewählt aus der Gruppe bestehend aus 1,2-Decandiol, Ethylhexylglycerin, 1,2-Octandiol, 1 ,2-Hexandiol, Glycerylcaprylat und Glycerylcaproat, und die Zusammensetzung neben der einen oder den mehreren Verbindungen der Formel (I) eine oder mehrere Verbindungen ausgewählt aus der Gruppe bestehend aus Sorbitol, Sorbitolestern, Sorbitan, Sorbitanestern, Isosorbid, Isosorbiddiestern und Carbonsäuren enthält, wobei die Carbonsäuren selbst als auch die den Säurekomponenten der genannten Ester zu Grunde liegenden Carbonsäuren der Formel RCOOH entsprechen, worin R die bei Formel (I) angegebene Bedeutung besitzt, und die OH-Zahl der Mischung aus der einen oder der mehreren Verbindungen der Formel (I) und der einen oder der mehreren Verbindungen ausgewählt aus der Gruppe bestehend aus Sorbitol, Sorbitolestern, Sorbitan, Sorbitanestern, Isosorbid, Isosorbiddiestern und Carbonsäuren kleiner oder gleich 245 ist.

Diese Zusammensetzungen sind somit Gegenstand der Erfindung.

Die erfindungsgemäßen Zusammensetzungen weisen eine sehr gute Konservierungsmittelleistung auf oder besitzen eine sehr gute Stabilität gegen mikrobiellen Befall, insbesondere gegen Hefen und Pilze, und basieren auf Grund des Vorhandenseins der Verbindungen der Formel (I) auch auf nachwachsenden Rohstoffen. Da die Verbindungen der Formel (I) die konservierende Wirkung von aliphatischen vicinalen Diolen sogar häufig synergistisch erhöhen, insbesondere gegen Hefen und Pilze, kann die Einsatzkonzentration der oftmals auf synthetischen Rohstoffen basierenden aliphatischen vicinalen Diole unter Erhalt einer hervorragenden antimikrobiellen Wirkung der erfindungsgemäßen Zusammensetzungen oder einer hervorragenden Stabilität der erfindungsgemäßen Zusammensetzungen gegen mikrobiellen Befall, insbesondere gegen Hefen und Pilze, signifikant verringert werden.

Im Vergleich zur Verwendung von organischen Säuren als Konservierungsmittel weisen die erfindungsgemäßen Zusammensetzungen zudem den Vorteil einer Wirksamkeit oder Stabilität gegen mikrobiellen Befall, insbesondere

gegen Hefen und Pilze, über einen breiteren pH-Wert-Bereich auf. Während organische Säuren oftmals eine gute Wirkung nur im pH-Wert-Bereich von 3,5 bis 6 zeigen, können die erfindungsgemäßen Zusammensetzungen auch bei höheren pH-Werten vorteilhaft eingesetzt werden.

Zusammensetzungen, beispielsweise kosmetische, dermatologische oder pharmazeutische Zusammensetzungen, enthaltend Ester auf Basis von nachwachsenden Rohstoffen sind bereits bekannt.

WO 2010/108738 A2 (Evonik) beschreibt Formulierungen zur Reinigung und Pflege von menschlichen oder tierischen Körperteilen enthaltend Sorbitancarbonsäureester, wobei sich der Carbonsäureanteil des Sorbitancarbonsäureesters ableitet von einer Carbonsäure enthaltend 6 bis 10 Kohlenstoffatome und die Sorbitancarbonsäureester eine Hydroxylzahl (OH-Zahl) von größer 350 aufweisen sowie die Verwendung der genannten Sorbitancarbonsäureester als Viskositätsregler, Pflegewirkstoff, Schaumbooster oder Solubilisator in reinigenden oder pflegenden Formulierungen.

DE 10 2009 022 444 (Clariant) beschreibt flüssige Zusammensetzungen enthaltend Sorbitanmonocaprylat und weitere antimikrobielle Wirkstoffe, sowie ihre Verwendung zum Konservieren von kosmetischen, dermatologischen oder pharmazeutischen Produkten.

In der DE 10 2009 022 445 (Clariant) werden flüssige Zusammensetzungen enthaltend Sorbitanmonocaprylat und Alkohol wie z. B. Ethylhexylglycerin und ihre Verwendung zum Konservieren von kosmetischen, dermatologischen oder pharmazeutischen Produkten offenbart.

JP 8173787 (A) (Lion) beschreibt eine Zusammensetzung enthaltend einen oberflächenaktiven Stoff enthaltend einen Fettsäureester von dehydratisiertem Sorbitol und die Verwendung als Öl-in-Wasser-Emulgator und als Reinigungsbasis. Die Zusammensetzungen können Mono- oder Diester von Capryl- und/oder Caprinsäure mit einem Polyol ausgewählt aus der Gruppe bestehend aus 1,5-Sorbitan, 1,4-Sorbitan und Isosorbid enthalten.

In der JP 8187070 (A) (Lion) wird eine Mischung aus Fettsäuremonoestern von $C_8$-$C_{18}$ Fettsäuren und mindestens einem Polyol ausgewählt aus Sorbitol, 1,5-Sorbitan, 1,4-Sorbitan und Isosorbid und Fettsäurediestern dieser Fettsäuren und Polyole in einem Gewichtsverhältnis von Monoester : Diester von 33 : 7 bis 9 : 1 als antimikrobieller Wirkstoff gegen Bakterien für Nahrungsmittel oder Getränke offenbart.

Verbindungen der Komponenten a) und b) der erfindungsgemäßen Zusammensetzungen sind käuflich erwerbbar oder können nach dem Fachmann geläufigen Methoden hergestellt werden. Beispielsweise können die Verbindungen der Formel (I) durch Veresterung von Isosorbid nach üblichen und dem Fachmann bekannten Methoden hergestellt werden, wobei sowohl Isosorbid selbst als auch die zur Veresterung verwendeten Säurekomponenten wiederum käuflich erwerbbar sind.

Der Rest R in der einen oder den mehreren Verbindungen der Formel (I) ist ein linearer gesättigter Alkylrest mit 7 bis 9 Kohlenstoffatomen.

Besonders bevorzugt ist der Rest R in der einen oder den mehreren Verbindungen der Formel (I) ein linearer gesättigter Alkylrest mit 7 Kohlenstoffatomen.

In Anspruch 1 ist die eine oder sind die mehreren Substanzen der Komponente b) ausgewählt aus der Gruppe bestehend aus 1,2-Decandiol, Ethylhexylglycerin, 1,2-Octandiol, 1,2-Hexandiol, Glycerylcaprylat und Glycerylcaproat.

Insbesondere bevorzugt ist die eine oder sind die mehreren Substanzen der Komponente b) ausgewählt aus der Gruppe bestehend aus 1,2-Decandiol, Ethylhexylglycerin, 1,2-Octandiol und 1,2-Hexandiol.

Außerordentlich bevorzugt ist die Substanz der Komponente b) Ethylhexylglycerin.

Ganz besonders bevorzugt ist der Rest R in der einen oder den mehreren Verbindungen der Formel (I) ein linearer gesättigter Alkylrest mit 7 Kohlenstoffatomen und handelt es sich bei der Substanz der Komponente b) um Ethylhexylglycerin.

In einer weiteren bevorzugten Ausführungsform der Erfindung enthalten die erfindungsgemäßen Zusammensetzungen

I) 0,05 bis 0,7, vorzugsweise 0,1 bis 0,7 und besonders bevorzugt 0,2 bis 0,5 Gewichtsteile Isosorbid und
II) 0,1 bis 1,0, vorzugsweise 0,2 bis 1,0 und besonders bevorzugt 0,4 bis 0,8 Gewichtsteile Isosorbiddiester der Formel

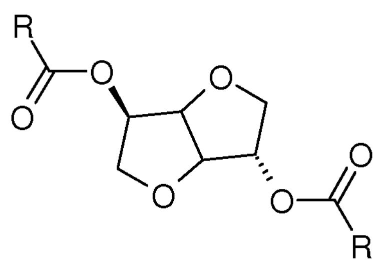

worin R die bei Formel (I) angegebene Bedeutung besitzt, und wobei der Isosorbiddiester vorzugsweise Isosorbiddicaprylat ist,
jeweils bezogen auf 1,0 Gewichtsteile an der einen oder den mehreren Verbindungen der Formel (I) und vorzugsweise bezogen auf 1,0 Gewichtsteile an Isosorbidmonocaprylat.

In einer hierunter wiederum bevorzugten Ausführungsform der Erfindung enthalten die erfindungsgemäßen Zusammensetzungen entweder keine Carbonsäure RCOOH oder bis zu 0,1, vorzugsweise 0,001 bis 0,05 und besonders bevorzugt 0,002 bis 0,01 Gewichtsteile Carbonsäure RCOOH, wobei R die bei Formel (I) angegebene Bedeutung besitzt, und wobei die Carbonsäure vorzugsweise Caprylsäure ist, bezogen auf 1,0 Gewichtsteile an der einen oder den mehreren Verbindungen der Formel (I) und vorzugsweise bezogen auf 1,0 Gewichtsteile an Isosorbidmonocaprylat.

In einer weiteren bevorzugten Ausführungsform der Erfindung enthalten die erfindungsgemäßen Zusammensetzungen zusätzlich einen oder mehrere Sorbitanester aus Sorbitan und Carbonsäuren $R^aCOOH$, vorzugsweise ausgewählt aus Sorbitanestern aus 1,4- und/oder 1,5-Sorbitan und Carbonsäuren $R^aCOOH$, wobei $R^a$ eine lineare oder verzweigte, gesättigte Alkylgruppe mit 5 bis 11, vorzugsweise 7 bis 9 und besonders bevorzugt 7 Kohlenstoffatomen oder eine lineare oder verzweigte, ein- oder mehrfach ungesättigte Alkenylgruppe mit 5 bis 11, vorzugsweise 7 bis 9 und besonders bevorzugt 7 Kohlenstoffatomen ist, und worin das Gewichtsverhältnis der einen oder der mehreren Verbindungen der Formel (I) zu dem einen oder den mehreren soeben genannten Sorbitanestern von 70 : 30 bis 100 : 0, vorzugsweise von 80 : 20 bis 100 : 0, besonders bevorzugt von 90 : 10 bis 100 : 0 und insbesondere bevorzugt von 95 : 5 bis 100 : 0 ist. Das angegebene Gewichtsverhältnis von "100 : 0" bedeutet, dass die erfindungsgemäßen Zusammensetzungen in dieser bevorzugten Ausführungsform der Erfindung keinen Sorbitanester enthalten müssen.

Unter den soeben genannten erfindungsgemäßen Zusammensetzungen sind solche bevorzugt, worin der eine oder die mehreren Sorbitanester aus Sorbitan und Carbonsäuren $R^aCOOH$ ausgewählt sind aus Sorbitanestern aus Sorbitan und Caprylsäure und vorzugsweise ausgewählt sind aus Sorbitanestern aus 1,4- und/oder 1,5-Sorbitan und Caprylsäure und der Sorbitanester besonders bevorzugt Sorbitanmonocaprylat ist.

In diesen Zusammensetzungen ist die OH-Zahl der Mischung aus der einen oder den mehreren Verbindungen der Formel (I) und der einen oder den mehreren (gegebenenfalls enthaltenen) Sorbitanestern aus Sorbitan und Carbonsäuren $R^aCOOH$ vorzugsweise kleiner oder gleich 245 und außerordentlich bevorzugt kleiner oder gleich 225.

Die erfindungsgemäßen Zusammensetzungen neben der einen oder den mehreren Verbindungen der Formel (I) eine oder mehrere Verbindungen ausgewählt aus der Gruppe bestehend aus Sorbitol, Sorbitolestern (bei Sorbitolestern kann es sich um Mono-, Di-, Tri-, Tetra-, Penta- und/oder Hexaester handeln), Sorbitan, Sorbitanestern (bei Sorbitanestern kann es sich um Mono-, Di-, Tri- und/oder Tetraester handeln), Isosorbid, Isosorbiddiestern und Carbonsäuren. Bei "Sorbitan" kann es sich beispielsweise um 1,4- oder 1,5-Sorbitan handeln. Sowohl die Carbonsäuren selbst als auch die den Säurekomponenten der genannten Ester zu Grunde liegenden Carbonsäuren entsprechen der Formel RCOOH, worin R die bei Formel (I) angegebene Bedeutung besitzt und vorzugsweise ein linearer gesättigter Alkylrest mit 7 Kohlenstoffatomen ist, d.h. die Carbonsäure RCOOH vorzugsweise Caprylsäure ist. Die OH-Zahl der Mischung aus der einen oder der mehreren Verbindungen der Formel (I) und der einen oder der mehreren Verbindungen ausgewählt aus der Gruppe bestehend aus Sorbitol, Sorbitolestern, Sorbitan, Sorbitanestern, Isosorbid, Isosorbiddiestern und Carbonsäuren ist kleiner oder gleich 245 und insbesondere bevorzugt kleiner oder gleich 225.

In einer weiteren bevorzugten Ausführungsform der Erfindung enthalten die erfindungsgemäßen Zusammensetzungen keine Verbindungen ausgewählt aus Sorbitol und Sorbitolestern.

In einer weiteren bevorzugten Ausführungsform der Erfindung enthalten die erfindungsgemäßen Zusammensetzungen keine Verbindungen ausgewählt aus Sorbitan und Sorbitanestern.

In einer bevorzugten Ausführungsform der Erfindung enthalten die erfindungsgemäßen Zusammensetzungen die eine oder die mehreren Verbindungen der Komponente a) in Mengen von 10,0 bis 90,0 Gew.-%, vorzugsweise in Mengen von 20,0 bis 80,0 Gew.-%, besonders bevorzugt in Mengen von 30,0 bis 70,0 Gew.-% und insbesondere bevorzugt von 40,0 bis 60,0 Gew.-% und die eine oder die mehreren Substanzen der Komponente b) in Mengen von 10,0 bis 90,0 Gew.-%, vorzugsweise in Mengen von 20,0 bis 80,0 Gew.-%, besonders bevorzugt in Mengen von 30,0 bis 70,0 Gew.-% und insbesondere bevorzugt in Mengen von 40,0 bis 60,0 Gew.-%, jeweils bezogen auf das Gesamtgewicht der Zusammensetzung.

Bei den soeben genannten erfindungsgemäßen Zusammensetzungen, in denen die Verbindungen der Komponente a) und die Substanzen der Komponente b) in relativ hohen Mengen enthalten sind, kann es sich beispielsweise um Zusammensetzungen bzw. "Vormischungen" handeln, die z. B. zur Herstellung von kosmetischen, dermatologischen oder pharmazeutischen Zusammensetzungen, Pflanzenschutzformulierungen oder Wasch- oder Reinigungsmitteln verwendet werden können.

Sofern diese erfindungsgemäßen Zusammensetzungen bzw. Vormischungen eine oder mehrere Verbindungen ausgewählt aus Sorbitol und Sorbitolestern (wobei die der Säurekomponente dieser Ester zu Grunde liegende Carbonsäure vorzugsweise Caprylsäure ist) enthalten, sind diese Verbindungen gemeinsam vorzugsweise in einer Menge kleiner oder gleich 5,0 Gew.-%, besonders bevorzugt in einer Menge kleiner oder gleich 3,0 Gew.-%, insbesondere bevorzugt in einer Menge kleiner oder gleich 1,0 Gew.-% und außerordentlich bevorzugt in einer Menge kleiner oder

gleich 0,5 Gew.-% in den erfindungsgemäßen Zusammensetzungen enthalten, wobei die Angaben in Gew.-% jeweils auf das Gesamtgewicht der fertigen erfindungsgemäßen Zusammensetzung bzw. Vormischung bezogen sind.

Sofern diese erfindungsgemäßen Zusammensetzungen bzw. Vormischungen eine oder mehrere Verbindungen ausgewählt aus Sorbitan und Sorbitanestern (wobei die der Säurekomponente dieser Ester zu Grunde liegende Carbonsäure vorzugsweise Caprylsäure ist) enthalten, sind diese Verbindungen gemeinsam vorzugsweise in einer Menge kleiner oder gleich 20,0 Gew.-%, besonders bevorzugt in einer Menge kleiner oder gleich 10,0 Gew.-%, insbesondere bevorzugt in einer Menge kleiner oder gleich 5,0 Gew.-% und außerordentlich bevorzugt in einer Menge kleiner oder gleich 1,0 Gew.-% in den erfindungsgemäßen Zusammensetzungen enthalten, wobei die Angaben in Gew.-% jeweils auf das Gesamtgewicht der fertigen erfindungsgemäßen Zusammensetzung bzw. Vormischung bezogen sind.

Insbesondere in dem Fall, dass die erfindungsgemäßen Zusammensetzungen bzw. Vormischungen geringe Mengen an der einen oder den mehreren Substanzen der Komponente b) enthalten, können sie bei Raumtemperatur (25 °C) fest sein. Vorzugsweise sind die erfindungsgemäßen Zusammensetzungen bei Raumtemperatur (25 °C) jedoch flüssig. In einer darunter bevorzugten Ausführungsform der Erfindung enthalten diese erfindungsgemäßen Zusammensetzungen bzw. Vormischungen zusätzlich zu den Verbindungen der Komponente a) und den Substanzen der Komponente b) ein oder mehrere nicht-aromatische Alkohole, wobei diese vorzugsweise ausgewählt sind aus der Gruppe bestehend aus Ethanol, Propylenglykol, 1,3-Propandiol und Glycerin.

Vorzugsweise enthalten diese erfindungsgemäßen Zusammensetzungen bzw. Vormischungen mindestens 50 Gew.-% an der einen oder mehreren Verbindungen der Komponente a), bezogen auf das Gesamtgewicht dieser erfindungsgemäßen Zusammensetzungen bzw. Vormischungen ohne Berücksichtigung der einen oder der mehreren Verbindungen der Komponente b).

Der pH-Wert für diese erfindungsgemäßen Zusammensetzungen bzw. Vormischungen, gemessen als 5 Gew.-%ige Lösung in Ethanol/Wasser (Gewichtsverhältnis Ethanol : Wasser 1 : 1) ist vorzugsweise 2 bis 9, besonders bevorzugt 4 bis 8 und insbesondere bevorzugt 5,5 bis 7,5.

Ein weiterer Vorteil der erfindungsgemäßen Zusammensetzungen und insbesondere der soeben genannten erfindungsgemäßen Zusammensetzungen bzw. Vormischungen besteht darin, dass sie neben der sehr guten konservierenden Wirkung zudem eine vorteilhafte Wirkung als Verdicker zeigen.

Unter der Hydroxyl- oder OH-Zahl einer Substanz wird diejenige Menge KOH in mg verstanden, die der bei der Acetylierung von 1 g Substanz gebundenen Menge an Essigsäure äquivalent ist.

Geeignete Bestimmungsmethoden zur Ermittlung der OH-Zahl sind z. B. DGF C-V 17 a (53), Ph. Eur. 2.5.3 Method A und DIN 53240.

Im Rahmen der vorliegenden Erfindung werden die OH-Zahlen in Anlehnung an DIN 53240-2 bestimmt. Hierbei wird wie folgt vorgegangen: Es wird 1 g auf 0,1 mg genau von der homogenisierten zu messenden Probe eingewogen. 20,00 ml Acetylierungsgemisch (Acetylierungsgemisch: in 1 l Pyridin werden 50 ml Essigsäureanhydrid eingerührt) werden zugeben. Die Probe wird vollständig im Acetylierungsgemisch gelöst, gegebenenfalls unter Rühren und Erwärmen. 5 ml Katalysatorlösung (Katalysatorlösung: 2 g 4-Dimethylaminopyridin werden in 100 ml Pyridin gelöst) werden zugeben. Das Reaktionsgefäß wird verschlossen und 10 Minuten in das auf 55 °C vorgeheizte Wasserbad gestellt und dabei durchmischt. Die Reaktionslösung wird danach mit 10 ml vollentsalztem Wasser versetzt, das Reaktionsgefäß erneut verschlossen und nochmals im Schüttelwasserbad 10 Minuten abreagieren gelassen. Die Probe wird auf Raumtemperatur (25 °C) abgekühlt. Anschließend werden 50 ml 2-Propanol und 2 Tropfen Phenolphthalein zugeben. Diese Lösung wird mit Natronlauge (Natronlauge c = 0,5 mol/l) titriert (Va). Unter den gleichen Bedingungen, jedoch ohne Probeneinwaage wird der Wirkwert des Acetylierungsgemisches bestimmt (Vb).

Aus dem Verbrauch der Wirkwertbestimmung und der Titration der Probe wird die OH-Zahl (OHZ) nach folgender Formel berechnet:

$$OHZ = \frac{(Vb - Va) \cdot c \cdot t \cdot M}{E}$$

OHZ = Hydroxylzahl in mg KOH/g Substanz
Va = Verbrauch an Natronlauge in ml bei der Titration der Probe
Vb = Verbrauch an Natronlauge in ml bei der Titration des Wirkwertes
c = Stoffmengenkonzentration der Natronlauge in mol/l
t = Titer der Natronlauge
M = Molare Masse von KOH = 56,11 g/mol
E = Probeneinwaage in g

(Vb - Va) ist diejenige Menge der verwendeten Natronlauge in ml, die der bei der oben beschriebenen Acetylierung der zu messenden Probe gebundenen Menge an Essigsäure äquivalent ist.

Die soeben beschriebene Methode zur Bestimmung der OH-Zahl wird im Folgenden als "Methode OHZ-A" bezeichnet.

In einer weiteren bevorzugten Ausführungsform der Erfindung sind die erfindungsgemäßen Zusammensetzungen kosmetische, dermatologische oder pharmazeutische Zusammensetzungen, Pflanzenschutzformulierungen oder Wasch- und Reinigungsmittel.

Die erfindungsgemäßen kosmetischen, dermatologischen oder pharmazeutischen Zusammensetzungen, Pflanzenschutzformulierungen oder Wasch- und Reinigungsmittel können aus den erfindungsgemäßen Vormischungen hergestellt werden. Alternativ können sie aber auch durch eine getrennte Verwendung der einen oder der mehreren Verbindungen der Formel (I) und der einen oder der mehreren Substanzen der Komponente b) hergestellt werden.

In einer bevorzugten Ausführungsform der Erfindung handelt es sich bei den erfindungsgemäßen Zusammensetzungen um kosmetische, dermatologische oder pharmazeutische Zusammensetzungen. Diese kosmetischen, dermatologischen oder pharmazeutischen zusammensetzungen werden im Folgenden beschrieben.

Die erfindungsgemäßen kosmetischen, dermatologischen oder pharmazeutischen Zusammensetzungen enthalten die eine oder die mehreren Verbindungen der Komponente a) vorzugsweise in Mengen von 0,01 bis 10,0 Gew.-%, besonders bevorzugt in Mengen von 0,1 bis 5,0 Gew.-%, insbesondere bevorzugt in Mengen von 0,2 bis 3,0 Gew.-% und außerordentlich bevorzugt in Mengen von 0,5 bis 2,0 Gew.-% und die eine oder die mehreren Substanzen der Komponente b) vorzugsweise in Mengen von 0,01 bis 10,0 Gew.-%, besonders bevorzugt in Mengen von 0,1 bis 5,0 Gew.-%, insbesondere bevorzugt in Mengen von 0,2 bis 3,0 Gew.-% und außerordentlich bevorzugt in Mengen von 0,5 bis 2,0 Gew.-%, jeweils bezogen auf das Gesamtgewicht der erfindungsgemäßen Zusammensetzung.

Wie bereits erwähnt enthalten die erfindungsgemäßen kosmetischen, dermatologischen oder pharmazeutischen Zusammensetzungen in einer bevorzugten Ausführungsform der Erfindung keine Verbindungen ausgewählt aus Sorbitol und Sorbitolestern. Sofern die erfindungsgemäßen kosmetischen, dermatologischen oder pharmazeutischen Zusammensetzungen jedoch eine oder mehrere Verbindungen ausgewählt aus Sorbitol und Sorbitolestern (wobei die der Säurekomponente dieser Ester zu Grunde liegende Carbonsäure vorzugsweise Caprylsäure ist) enthalten, sind diese Verbindungen gemeinsam vorzugsweise in einer Menge kleiner oder gleich 0,1 Gew.-%, besonders bevorzugt in einer Menge kleiner oder gleich 0,06 Gew.-%, insbesondere bevorzugt in einer Menge kleiner oder gleich 0,02 Gew.-% und außerordentlich bevorzugt in einer Menge kleiner oder gleich 0,01 Gew.-% in den erfindungsgemäßen kosmetischen, dermatologischen oder pharmazeutischen Zusammensetzungen enthalten, wobei die Angaben in Gew.-% jeweils auf das Gesamtgewicht der fertigen erfindungsgemäßen Zusammensetzung bezogen sind.

Wie bereits erwähnt enthalten die erfindungsgemäßen kosmetischen, dermatologischen oder pharmazeutischen Zusammensetzungen in einer weiteren bevorzugten Ausführungsform der Erfindung keine Verbindungen ausgewählt aus Sorbitan und Sorbitanestern. Sofern die erfindungsgemäßen kosmetischen, dermatologischen oder pharmazeutischen Zusammensetzungen jedoch eine oder mehrere Verbindungen ausgewählt aus Sorbitan und Sorbitanestern (wobei die der Säurekomponente dieser Ester zu Grunde liegende Carbonsäure vorzugsweise Caprylsäure ist) enthalten, sind diese Verbindungen gemeinsam vorzugsweise in einer Menge kleiner oder gleich 0,4 Gew.-%, besonders bevorzugt in einer Menge kleiner oder gleich 0,2 Gew.-%, insbesondere bevorzugt in einer Menge kleiner oder gleich 0,1 Gew.-% und außerordentlich bevorzugt in einer Menge kleiner oder gleich 0,02 Gew.-% in den erfindungsgemäßen kosmetischen, dermatologischen oder pharmazeutischen Zusammensetzungen enthalten, wobei die Angaben in Gew.-% jeweils auf das Gesamtgewicht der fertigen erfindungsgemäßen Zusammensetzung bezogen sind.

In einer weiteren bevorzugten Ausführungsform der Erfindung haben die erfindungsgemäßen kosmetischen, dermatologischen oder pharmazeutischen Zusammensetzungen Viskositäten vorzugsweise im Bereich von 50 bis 200 000 mPa · s, besonders bevorzugt im Bereich von 500 bis 100 000 mPa · s, insbesondere bevorzugt im Bereich von 2 000 bis 50 000 mPa · s und außerordentlich bevorzugt im Bereich von 5 000 bis 30 000 mPa · s (20 °C, Brookfield RVT, RV-Spindel-Satz bei 20 Umdrehungen pro Minute).

In einer weiteren bevorzugten Ausführungsform der Erfindung liegen die erfindungsgemäßen kosmetischen, dermatologischen oder pharmazeutischen Zusammensetzungen in Form von Fluids, Gelen, Schäumen, Sprays, Lotions oder Cremes vor.

Die erfindungsgemäßen kosmetischen, dermatologischen oder pharmazeutischen Zusammensetzungen sind vorzugsweise auf wässriger oder wässrig-alkoholischer Basis aufgebaut oder liegen als Lösungen, Emulsionen oder Dispersionen vor. Besonders bevorzugt liegen sie als Emulsionen vor und insbesondere bevorzugt liegen sie als Öl-in-Wasser Emulsionen vor.

In einer besonders bevorzugten Ausführungsform der Erfindung liegen die erfindungsgemäßen kosmetischen, dermatologischen oder pharmazeutischen Zusammensetzungen als Öl-in-Wasser Emulsionen vor und enthalten vorzugsweise, bezogen auf das Gesamtgewicht der Zusammensetzungen,

a) bis zu 95,0 Gew.-%, vorzugsweise 49,49 bis 95,0 Gew.-%, besonders bevorzugt 68,9 bis 90,0 Gew.-%, insbesondere bevorzugt 70,0 bis 85,0 Gew.-% einer Wasserphase oder wässrigen-alkoholischen Phase,

b) bis zu 70,0 Gew.-%, vorzugsweise 4,49 bis 50,0 Gew.-%, besonders bevorzugt 8,9 bis 30,0 Gew.-%, insbesondere bevorzugt 13,5 bis 25,0 Gew.-% einer Ölphase,

c) bis zu 10,0 Gew.-%, vorzugsweise 0,01 bis 10,0 Gew.-%, besonders bevorzugt 0,05 bis 5,0 Gew.-%, insbesondere bevorzugt 0,1 bis 2,0 Gew.-% an einer Zusammensetzung nach Anspruch 1, wobei die Zusammensetzung die eine oder mehrere Verbindungen nach der Formel (I) und ein oder mehrere aliphatische vicinale Diole in einer Menge von vorzugsweise 30 Gew.-% oder mehr, besonders bevorzugt 40 Gew.-% oder mehr und insbesondere bevorzugt 50 Gew.-% oder mehr enthält, wobei weiterhin das Gewichtsverhältnis Verbindungen der Formel (I) : aliphatischen vicinalen Diolen vorzugsweise von 90,0 : 10,0 bis 10,0 : 90,0 und besonders bevorzugt von 80,0 : 20,0 bis 20,0 : 80,0 ist und wobei es sich bei der Zusammensetzung weiterhin bevorzugt um eine erfindungsgemäße Vormischung handelt und

d) bis zu 20,0 Gew.-%, vorzugsweise 0,5 bis 10 Gew.-%, besonders bevorzugt 1,0 bis 5,0 Gew.-%, insbesondere bevorzugt 1,0 bis 3,0 Gew.-% eines oder mehrerer weiterer Zusatzstoffe.

Vorzugsweise ist der eine oder sind die mehreren weiteren Zusatzstoffe in den soeben genannten Öl-in-Wasser Emulsionen ausgewählt aus der Gruppe bestehend aus Emulgatoren, Co-Emulgatoren, Solubilisatoren, Aktivstoffen, Sonnenschutzfiltern, Pigmenten und antimikrobiellen Wirkstoffen.

Für die erfindungsgemäßen kosmetischen, dermatologischen oder pharmazeutischen Zusammensetzungen auf wässrig-alkoholischer oder auch alkoholischer Basis kommen alle ein- oder mehrwertigen Alkohole in Betracht. Vorzugsweise werden Alkohole mit 1 bis 4 Kohlenstoffatomen wie Ethanol, Propanol, Isopropanol, n-Butanol, i-Butanol, tert.-Butanol oder Glycerin sowie Alkylenglykole, insbesondere Propylen- oder Butylenglykol, und Mischungen aus den genannten Alkoholen verwendet. Weitere bevorzugte Alkohole sind Polyethylenglykole mit einer relativen Molekülmasse unter 2 000. Besonders bevorzugt ist ein Einsatz von Ethanol oder Isopropanol.

Die erfindungsgemäßen kosmetischen, dermatologischen oder pharmazeutischen Zusammensetzungen können ein oder mehrere Öle enthalten.

Die Öle können vorteilhafterweise ausgewählt werden aus den Gruppen der Triglyceride, natürlichen und synthetischen Fettkörper, vorzugsweise Ester von Fettsäuren mit Alkoholen niedriger C-Zahl, z. B. mit Methanol, Isopropanol oder Propylenglykol, oder Ester von Fettalkoholen mit Alkansäuren niedriger C-Zahl oder mit Fettsäuren oder aus der Gruppe der Alkylbenzoate, sowie natürlichen oder synthetischen Kohlenwasserstoffölen.

In Betracht kommen Triglyceride von linearen oder verzweigten, gesättigten oder ungesättigten, gegebenenfalls hydroxylierten, $C_8$-$C_{30}$-Fettsäuren, insbesondere pflanzliche Öle, wie Sonnenblumen-, Mais-, Soja-, Reis-, Jojoba-, Babusscu-, Kürbis-, Traubenkern-, Sesam-, Walnuss-, Aprikosen-, Orangen-, Weizenkeim-, Pfirsichkern-, Makadamia-, Avocado-, Süßmandel-, Wiesenschaumkraut-, Ricinusöl, Olivenöl, Erdnussöl, Rapsöl und Kokosnussöl, sowie synthetische Triglyceridöle, z. B. das Handelsprodukt Myritol® 318. Auch gehärtete Triglyceride kommen in Betracht. Auch Öle tierischen Ursprungs, beispielsweise Rindertalg, Perhydrosqualen, Lanolin können eingesetzt werden.

Eine weitere Klasse von bevorzugten Ölkörpern sind die Benzoesäureester von linearen oder verzweigten $C_{8-22}$-Alkanolen, z. B. die Handelsprodukte Finsolv®SB (Isostearylbenzoat), Finsolv®TN ($C_{12}$-$C_{15}$-Alkylbenzoat) und Finsolv®EB (Ethylhexylbenzoat).

Eine weitere Klasse von bevorzugten Ölkörpern sind die Dialkylether mit insgesamt 12 bis 36 Kohlenstoffatomen, insbesondere mit 12 bis 24 Kohlenstoffatomen, wie z. B. Di-n-octylether (Cetiol® OE), Di-n-nonylether, Di-n-decylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-undecylether, Di-3-ethyldecylether, tert.-Butyl-n-octylether, iso-Pentyl-n-octylether und 2-Methyl-pentyl-n-octylether sowie Di-tert.-butylether und Di-iso-pentylether.

Ebenso in Betracht kommen verzweigte gesättigte oder ungesättigte Fettalkohole mit 6 - 30 Kohlenstoffatomen, z. B. Isostearylalkohol, sowie Guerbetalkohole.

Eine weitere Klasse von bevorzugten Ölkörpern sind Hydroxycarbonsäurealkylester. Bevorzugte Hydroxycarbonsäurealkylester sind Vollester der Glykolsäure, Milchsäure, Apfelsäure, Weinsäure oder Zitronensäure. Weitere grundsätzlich geeignete Ester der Hydroxycarbonsäuren sind Ester der β-Hydroxypropionsäure, der Tartronsäure, der D-Gluconsäure, Zuckersäure, Schleimsäure oder Glucuronsäure. Als Alkoholkomponente dieser Ester eignen sich primäre, lineare oder verzweigte aliphatische Alkohole mit 8 bis 22 C-Atomen. Dabei sind die Ester von $C_{12}$-$C_{15}$-Fettalkoholen besonders bevorzugt. Ester dieses Typs sind im Handel erhältlich, z. B. unter dem Handelsnamen Cosmacol® der EniChem, Augusta Industriale.

Eine weitere Klasse von bevorzugten Ölkörpern sind Dicarbonsäureester von linearen oder verzweigten $C_2$-$C_{10}$-Alkanolen, wie Di-n-butyladipat (Cetiol® B), Di-(2-ethylhexyl)-adipat und Di-(2-ethylhexyl)-succinat sowie Diolester wie Ethylenglycol-dioleat, Ethylenglycol-di-isotridecanoat, Propylenglycol-di-(2-ethylhexanoat), Propylenglycol-di-isostearat, Propylenglycol-di-pelargonat, Butandiol-di-isostearat und Neopentylglycoldicaprylat sowie Di-isotridecylacelaat.

Ebenso bevorzugte Ölkörper sind symmetrische, unsymmetrische oder cyclische Ester der Kohlensäure mit Fettalkoholen, Glycerincarbonat oder Dicaprylylcarbonat (Cetiol® CC).

Eine weitere Klasse von bevorzugten Ölkörpern sind die Ester von Dimeren ungesättigter $C_{12}$-$C_{22}$-Fettsäuren (Dimerfettsäuren) mit einwertigen linearen, verzweigten oder cyclischen $C_2$-$C_{18}$-Alkanolen oder mit mehrwertig linearen oder verzweigten $C_2$-$C_6$-Alkanolen.

Eine weitere Klasse von bevorzugten Ölkörpern sind Kohlenwasserstofföle, zum Beispiel solche mit linearen oder verzweigten, gesättigten oder ungesättigten $C_7$-$C_{40}$-Kohlenstoffketten, beispielsweise Vaseline, Dodecan, Isododecan, Cholesterol, Lanolin, synthetische Kohlenwasserstoffe wie Polyolefine, insbesondere Polyisobuten, hydriertes Polyisobuten, Polydecan, sowie Hexadecan, Isohexadecan, Paraffinöle, Isoparaffinöle, z. B. die Handelsprodukte der Permethyl®-Serie, Squalan, Squalen, und alicyclische Kohlenwasserstoffe, z. B. das Handelsprodukt 1,3-Di-(2-ethylhexyl)-cyclohexan (Cetiol® S), Ozokerit, und Ceresin.

Ebenso in Betracht kommen Silikonöle bzw. -wachse, vorzugsweise Dimethylpolysiloxane und Cyclomethicone, Polydialkylsiloxane $R_3SiO(R_2SiO)_xSiR_3$, wobei R für Methyl oder Ethyl, besonders bevorzugt für Methyl, steht und x für eine Zahl von 2 bis 500 steht, beispielsweise die unter den Handelsnamen VICASIL (General Electric Company), DOW CORNING 200, DOW CORNING 225, DOW CORNING 200 (Dow Corning Corporation), erhältlichen Dimethicone, sowie die unter SilCare® Silicone 41 M65, SilCare® Silicone 41M70, SilCare® Silicone 41M80 (Clariant) erhältlichen Dimethicone, Stearyldimethylpolysiloxan, $C_{20}$-$C_{24}$-Alkyl-dimethylpolysiloxan, $C_{24}$-$C_{28}$-Alkyl-dimethylpolysiloxan, aber auch die unter SilCare® Silicone 41M40, SilCare® Silicone 41M50 (Clariant) erhältlichen Methicone, weiterhin Trimethylsiloxysilicate $[(CH_2)_3SiO)_{1/2}]x[SiO_2]_y$, wobei x für eine Zahl von 1 bis 500 und y für eine Zahl von 1 bis 500 steht, Dimethiconole $R_3SiO[R_2SiO]_xSiR_2OH$ und $HOR_2SiO[R_2SiO]_xSiR_2OH$, wobei R für Methyl oder Ethyl und x für eine Zahl bis zu 500 steht, Polyalkylarylsiloxane, beispielsweise die unter den Handelsbezeichnungen SF 1075 METHYLPHENYL FLUID (General Electric Company) und 556 COSMETIC GRADE PHENYL TRIMETHICONE FLUID (Dow Corning Corporation) erhältlichen Polymethylphenylsiloxane, Polydiarylsiloxane, Silikonharze, cyclische Silikone und amino-, fettsäure-, alkohol-, polyether-, epoxy-, fluor- und/oder alkylmodifizierte Silikonverbindungen, sowie Polyethersiloxan-Copolymere.

Die erfindungsgemäßen kosmetischen, dermatologischen oder pharmazeutischen Zusammensetzungen können als weitere Hilfs- und Zusatzstoffe beispielsweise Wachse, Emulgatoren, Co-Emulgatoren, Solubilisatoren, Elektrolyte, Hydroxysäuren, Stabilisatoren, kationische Polymere, Filmbildner, weitere Verdicker, Gelierungsmittel, Überfettungsmittel, Rückfetter, weitere antimikrobielle Wirkstoffe, biogene Wirkstoffe, Adstringentien, deodorierende Stoffe, Sonnenschutzfilter, Antioxidantien, Feuchthaltemittel, Lösungsmittel, Farbmittel, Perlglanzmittel, Duftstoffe, Trübungsmittel und/oder Silikone enthalten.

Die erfindungsgemäßen kosmetischen, dermatologischen oder pharmazeutischen Zusammensetzungen können Wachse, beispielsweise Paraffinwachse, Mikrowachse und Ozokerite, Bienenwachs und ihre Teilfraktionen sowie Bienenwachsderivate, Wachse aus der Gruppe der homopolymeren Polyethylene oder Coplymere der $\alpha$-Olefine, sowie natürliche Wachse wie Reiswachs, Candellilawachs, Carnaubawachs, Japanwachs oder Schellackwachs enthalten.

Als Emulgatoren, Co-Emulgatoren und Solubilisatoren können nichtionische, anionische, kationische oder amphotere oberflächenaktive Verbindungen eingesetzt werden.

Als nichtionogene oberflächenaktive Verbindungen kommen vorzugsweise in Betracht:

Anlagerungsprodukte von 1 bis 30 Mol Ethylenoxid und/oder 1 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe und an Sorbitan- bzw. Sorbitolester; ($C_{12}$-$C_{18}$)-Fettsäuremono- und -diester von Anlagerungsprodukten von 1 bis 30 Mol Ethylenoxid an Glycerin; Glycerinmono- und -diester und Sorbitanmono- und -diester von gesättigten und ungesättigten Fettsäuren mit 14 bis 22 Kohlenstoffatomen und gegebenenfalls deren Ethylenoxidanlagerungsprodukten; Anlagerungsprodukte von 15 bis 60 Mol Ethylenoxid an Rizinusöl und/oder gehärtetes Rizinusöl; Polyol- und insbesondere Polyglycerinester, wie z. B. Polyglycerinpolyricinoleat und Polyglycerinpoly-12-hydroxystearat. Ebenfalls vorzugsweise geeignet sind ethoxylierte Fettamine, Fettsäureamide, Fettsäurealkanolamide und Gemische von Verbindungen aus mehreren dieser Substanzklassen.

Als ionogene Co-Emulgatoren eignen sich z. B. anionische Emulgatoren, wie mono-, di- oder tri-Phosphorsäureester, Seifen (z. B. Natriumstearat), Fettalkoholsulfate aber auch kationische Emulgatoren wie mono-, di- und tri-Alkylquats und deren polymere Derivate.

An amphoteren Emulgatoren stehen vorzugsweise zur Verfügung Alkylaminoalkylcarbonsäuren, Betaine, Sulfobetaine und Imidazolinderivate.

Besonders bevorzugt zum Einsatz kommen Fettalkoholethoxylate, gewählt aus der Gruppe der ethoxylierten Stearylalkohole, Isostearylalkohole, Cetylalkohole, Isocetylalkohole, Oleylalkohole, Laurylalkohole, Isolaurylalkohole und Cetylstearylalkohole, insbesondere Polyethylenglykol(13)stearylether, Polyethylenglykol(14)stearylether, Polyethylenglykol(15)stearylether, Polyethylenglykol(16)stearylether, Polyethylenglykol(17)stearylether, Polyethylenglykol(18)stearylether, Polyethylenglykol(19)stearylether, Polyethylenglykol(20)stearylether, Polyethylenglykol(12)isostearylether, Polyethylenglykol(13)isostearylether, Polyethylenglykol(14)isostearylether, Polyethylenglykol(15)isostearylether, Polyethylenglykol(16)isostearylether, Polyethylenglykol(17)isostearylether, Polyethylenglykol(18)isostearylether,

Polyethylenglykol(19)isostearylether, Polyethylenglykol(20)isostearylether, Polyethylenglykol(13)cetylether, Polyethylenglykol(14)cetylether, Polyethylenglykol(15)cetylether, Polyethylenglykol(16)cetylether, Polyethylenglykol(17)cetylether, Polyethylenglykol(18)cetylether, Polyethylenglykol(19)cetylether, Polyethylenglykol(20)cetylether, Polyethylenglykol(13)isocetylether, Polyethylenglykol(14)isocetylether, Polyethylenglykol(15)isocetylether, Polyethylenglykol(16)isocetylether, Polyethylenglykol(17)isocetylether, Polyethylenglykol(18)isocetylether, Polyethylenglykol(19)isocetylether, Polyethylenglykol(20)isocetylether, Polyethylenglykol(12)oleylether, Polyethylenglykol(13)oleylether, Polyethylenglykol(14)oleylether, Polyethylenglykol(15)oleylether, Polyethylenglykol(12)laurylether, Polyethylenglykol(12)isolaurylether, Polyethylenglykol(13)cetylstearylether, Polyethylenglykol(14)cetylstearylether, Polyethylenglykol(15)cetylstearylether, Polyethylenglykol(16)cetylstearylether, Polyethylenglykol(17)cetylstearylether, Polyethylenglykol(18)cetylstearylether, Polyethylenglykol(19)cetylstearylether.

Ebenso bevorzugt sind Fettsäureethoxylate, gewählt aus der Gruppe der ethoxylierten Stearate, Isostearate und Oleate, insbesondere Polyethylenglycol(20)stearat, Polyethylenglykol(21)stearat, Polyethylenglykol(22)stearat, Polyethylenglykol(23)stearat, Polyethylenglykol(24)stearat, Polyethylenglykol(25)stearat, Polyethylenglykol(12)isostearat, Polyethylenglykol(13)isostearat, Polyethylenglykol(14)isostearat, Polyethylenglykol(15)isostearat, Polyethylenglykol(16)isostearat, Polyethylenglykol(17)isostearat, Polyethylenglykol(18)isostearat, Polyethylenglykol(19)isostearat, Polyethylenglykol(20)isostearat, Polyethylenglykol(21)isostearat, Polyethylenglykol(22)isostearat, Polyethylenglykol(23)isostearat, Polyethylenglykol(24)isostearat, Polyethylenglykol(25)isostearat, Polyethylenglykol(12)oleat, Polyethylenglykol(13)oleat, Polyethylenglykol(14)oleat, Polyethylenglykol(15)oleat, Polyethylenglykol(16)oleat, Polyethylenglykol(17)oleat, Polyethylenglykol(18)oleat, Polyethylenglykol(19)oleat, Polyethylenglykol(20)oleat.

Als ethoxylierte Alkylethercarbonsäure oder deren Salze kann vorteilhafterweise das Natrium Laureth-11-carboxylat verwendet werden.

Als ethoxylierte Triglyceride können vorteilhaft Polyethylenglykol(60)Evening Primose Glyceride verwendet werden.

Weiterhin ist es von Vorteil, die Polyethylenglykolglycerinfettsäureester aus der Gruppe Polyethylenglykol(20)glyceryllaurat, Polyethylenglykol(6)glycerylcaprat/caprinat, Polyethylenglykol(20)glyceryloleat, Polyethylenglykol(20)glycerylisostearat und Polyethylenglykol(18)glyceryloleat/cocoat zu wählen.

Unter den ethoxylierten Sorbitanestern eignen sich besonders Polyethylenglykol(20)sorbitanmonolaurat, Polyethylenglykol(20)sorbitanmonostearat, Polyethylenglykol(20)sorbitanmonoisostearat, Polyethylenglykol(20)sorbitanmonopalmitat, Polyethylenglykol(20)sorbitanmonooleat.

Besonders vorteilhafte Coemulgatoren sind Glycerylmonostearat, Glycerylmonooleat, Diglycerylmonostearat, Glycerylisostearat, Polyglyceryl-3-oleat , Polyglyceryl-3-diisostearat, Polyglyceryl-4-isostearat, Polyglyceryl-2-dipolyhydroxystearat, Polyglyceryl-4-dipolyhydroxystearat, PEG-30-dipolyhydroxystearat, Diisostearoylpolyglyceryl-3-diisostearat, Glycoldistearat und Polyglyceryl-3-dipolyhydroxystearat, Sorbitanmonoisostearat, Sorbitanstearat, Sorbitanoleat, Saccharosedistearat, Lecithin, PEG-7-hydriertes Ricinusöl, Cetylalkohol, Stearylalkohol, Behenylalkohol, Isobehenylalkohol und Polyethylenglykol(2)stearylether (Steareth-2), Alkylmethiconcopolyole und Alkyl-Dimethiconcopolyole, insbesondere Cetyldimethiconcopolyol (ABIL® EM 90), Laurylmethiconcopolyol oder Amodimethicone Glycerocarbamate (SilCare® Silicone WSI, Clariant).

Sofern die erfindungsgemäßen kosmetischen, dermatologischen oder pharmazeutischen Zusammensetzungen eine oder mehrere Substanzen ausgewählt aus der Gruppe bestehend aus Emulgatoren, Co-Emulgatoren und Solubilisatoren enthalten, ist diese eine oder sind diese mehreren Substanzen, bezogen auf das Gesamtgewicht der entsprechenden erfindungsgemäßen Zusammensetzung, vorzugsweise in einer Menge von 0,1 bis 20,0 Gew.-%, besonders bevorzugt in einer Menge von 0,5 bis 10,0 Gew.-% und insbesondere bevorzugt in einer Menge von 1,0 bis 5,0 Gew.-% in den erfindungsgemäßen Zusammensetzungen enthalten.

Als Elektrolyt zum Einsatz können kommen anorganische Salze, bevorzugt Ammonium- oder Metallsalze, besonders bevorzugt von Halogeniden, beispielsweise $CaCl_2$, $MgCl_2$, LiCl, KCl und NaCl, Carbonaten, Hydrogencarbonaten, Phosphaten, Sulfaten, Nitraten, insbesondere bevorzugt Natriumchlorid, und/oder organische Salze, bevorzugt Ammonium- oder Metallsalze, besonders bevorzugt der Glykolsäure, Milchsäure, Zitronensäure, Weinsäure, Mandelsäure, Salicylsäure, Ascorbinsäure, Brenztraubensäure, Fumarsäure, Retinoesäure, Sulfonsäuren, Benzoesäure, Kojisäure, Fruchtsäure, Äpfelsäure, Gluconsäure oder Galacturonsäure.

Hierzu zählen auch Aluminiumsalze, bevorzugt Aluminiumchlorohydrat oder Aluminium-Zirkonium-Komplexsalze.

In einer bevorzugten Ausführungsform der Erfindung enthalten die erfindungsgemäßen kosmetischen, dermatologischen oder pharmazeutischen Zusammensetzungen daher ein oder mehrere Substanzen ausgewählt aus anorganischen und organischen Salzen.

Als Elektrolyt können die erfindungsgemäßen kosmetischen, dermatologischen oder pharmazeutischen Zusammensetzungen auch Mischungen verschiedener Salze enthalten.

Sofern die erfindungsgemäßen kosmetischen, dermatologischen oder pharmazeutischen Zusammensetzungen einen oder mehrere Elektrolyte enthalten, sind diese, bezogen auf das Gesamtgewicht der entsprechenden erfindungsgemäßen Zusammensetzung, vorzugsweise in einer Menge von 0,01 bis 20,0 Gew.-%, besonders bevorzugt in

einer Menge von 0,1 bis 10,0 Gew.-% und insbesondere bevorzugt in einer Menge von 0,5 bis 5,0 Gew.-% in den erfindungsgemäßen Zusammensetzungen enthalten.

In einer weiteren bevorzugten Ausführungsform der Erfindung enthalten die erfindungsgemäßen kosmetischen, dermatologischen oder pharmazeutischen Zusammensetzungen eine oder mehrere Hydroxysäuren, besonders bevorzugt eine oder mehrere Substanzen ausgewählt aus alpha- und beta-Hydroxysäuren.

An Hydroxysäuren können die erfindungsgemäßen kosmetischen, dermatologischen oder pharmazeutischen Zusammensetzungen vorzugsweise Milchsäure, Glykolsäure, Salicylsäure und alkylierte Salicylsäuren oder Zitronensäure enthalten. Weiterhin können die erfindungsgemäßen kosmetischen, dermatologischen oder pharmazeutischen Zusammensetzungen weitere saure Komponenten enthalten. Als Wirkstoff in Betracht kommen Weinsäure, Mandelsäure, Caffeic acid, Brenztraubensäure, Oligooxa Mono- und Dicarbonsäuren, Fumarsäure, Retinoesäure, Sulfonsäuren, Benzoesäure, Kojisäure, Fruchtsäure, Äpfelsäure, Gluconsäure, Pyruvic Acid, Galacturonic Acid, Ribonic Acid, und all deren Derivate, Polyglykoldisäuren in freier oder teilweiser neutralisierter Form, Vitamin C (Ascorbinsäure), Vitamin C-Derivate, Dihydroxyaceton oder Skin-whitening Actives wie Arbutin oder Glycyrrhetinsäure und deren Salze. Sofern die erfindungsgemäßen kosmetischen, dermatologischen oder pharmazeutischen Zusammensetzungen eine oder mehrere dieser soeben genannten Substanzen enthalten, ist diese eine oder sind diese mehreren Substanzen, bezogen auf das Gesamtgewicht der entsprechenden erfindungsgemäßen Zusammensetzung, vorzugsweise in einer Menge von 0,1 bis 20,0 Gew.-%, besonders bevorzugt in einer Menge von 0,2 bis 10,0 Gew.-% und insbesondere bevorzugt in einer Menge von 0,5 bis 5,0 Gew.-% in den erfindungsgemäßen Zusammensetzungen enthalten.

In einer weiteren bevorzugten Ausführungsform der Erfindung enthalten die erfindungsgemäßen kosmetischen, dermatologischen oder pharmazeutischen Zusammensetzungen daher ein oder mehrere Substanzen ausgewählt aus Vitamin C und Vitamin C-Derivaten, wobei die Vitamin C-Derivate vorzugsweise ausgewählt sind aus Natriumascorbylphosphat, Magnesiumascorbylphoshat und Magnesiumascorbylglucosid.

In einer weiteren bevorzugten Ausführungsform der Erfindung enthalten die erfindungsgemäßen kosmetischen, dermatologischen oder pharmazeutischen Zusammensetzungen eine oder mehrere Substanzen ausgewählt aus der Gruppe bestehend aus Benzoesäure, Sorbinsäure, Salicylsäure, Milchsäure und Paramethoxybenzoesäure. Die oben genannten organischen Säuren können als weitere Konservierungsmittel dienen.

Als Stabilisatoren können in den erfindungsgemäßen kosmetischen, dermatologischen oder pharmazeutischen Zusammensetzungen Metallsalze von Fettsäuren, wie z. B. Magnesium-, Aluminium- und/oder Zinkstearat eingesetzt werden. Sofern die erfindungsgemäßen kosmetischen, dermatologischen oder pharmazeutischen Zusammensetzungen eine oder mehrere dieser soeben genannten Substanzen enthalten, ist diese eine oder sind diese mehreren Substanzen, bezogen auf das Gesamtgewicht der entsprechenden erfindungsgemäßen Zusammensetzung, vorzugsweise in einer Menge von 0,1 bis 10,0 Gew.-%, besonders bevorzugt in einer Menge von 0,5 bis 8,0 Gew.-% und insbesondere bevorzugt in einer Menge von 1,0 bis 5,0 Gew.-% in den erfindungsgemäßen Zusammensetzungen enthalten.

Als kationische Polymere eignen sich die unter der INCI-Bezeichnung "Polyquaternium" bekannten Substanzen, insbesondere Polyquaternium-31, Polyquaternium-16, Polyquaternium-24, Polyquaternium-7, Polyquaternium-22, Polyquaternium-39, Polyquaternium-28, Polyquaternium-2, Polyquaternium-10, Polyquaternium-11, sowie Polyquaternium 37&mineral oil&PPG trideceth (Salcare SC95), PVP-dimethylaminoethylmethacrylat-Copolymer, Guarhydroxypropyltriammonium-chloride, sowie Calciumalginat und Ammoniumalginat. Des Weiteren können eingesetzt werden kationische Cellulosederivate; kationische Stärke; Copolymere von Diallylammoniumsalzen und Acrylamiden; quaternierte Vinylpyrrolidon/Vinylimidazol-Polymere; Kondensationsprodukte von Polyglykolen und Aminen; quaternierte Kollagenpolypeptide; quaternierte Weizenpolypeptide; Polyethylenimine; kationische Siliconpolymere, wie z. B. Amidomethicone; Copolymere der Adipinsäure und Dimethylaminohydroxypropyldiethylentriamin; Polyaminopolyamid und kationische Chitinderivate, wie beispielsweise Chitosan.

Sofern die erfindungsgemäßen kosmetischen, dermatologischen oder pharmazeutischen Zusammensetzungen ein oder mehrere der oben genannten kationischen Polymere enthalten, sind diese, bezogen auf das Gesamtgewicht der entsprechenden erfindungsgemäßen Zusammensetzung, vorzugsweise in einer Menge von 0,1 bis 5,0 Gew.-%, besonders bevorzugt in einer Menge von 0,2 bis 3,0 Gew.-% und insbesondere bevorzugt in einer Menge von 0,5 bis 2,0 Gew.-% in den erfindungsgemäßen Zusammensetzungen enthalten.

Des Weiteren können die erfindungsgemäßen kosmetischen, dermatologischen oder pharmazeutischen Zusammensetzungen Filmbildner enthalten, die je nach Anwendungszweck ausgewählt sind aus Salzen der Phenylbenzimidazolsulfonsäure, wasserlöslichen Polyurethanen, beispielsweise $C_{10}$-Polycarbamylpolyglycerylester, Polyvinylalkohol, Polyvinylpyrrolidoncopolymeren, beispielsweise Vinylpyrrolidon/Vinylacetatcopolymer oder PVP/Eicosene-Copolymeren, maleinierten Polypropylenpolymeren, wasserlöslichen Acrylsäurepolymeren/Copolymeren bzw. deren Estern oder Salzen, beispielsweise Partialestercopolymere der Acryl/Methacrylsäure, wasserlöslicher Cellulose, beispielsweise Hydroxymethylcellulose, Hydroxyethylcellulose, Hydroxypropylcellulose, wasserlöslichen Quaterniums, Polyquaterniums, Carboxyvinyl-Polymeren, wie Carbomere und deren Salze, Polysacchariden, beispielsweise Polydextrose und Glucan, Vinylacetat/Crotonat, beispielsweise unter dem Handelsnamen Aristoflex® A 60 (Clariant) erhältlich.

Sofern die erfindungsgemäßen kosmetischen, dermatologischen oder pharmazeutischen Zusammensetzungen einen oder mehrere Filmbildner enthalten, sind diese, bezogen auf das Gesamtgewicht der entsprechenden erfindungsgemäßen Zusammensetzung, vorzugsweise in einer Menge von 0,1 bis 10,0 Gew.-%, besonders bevorzugt in einer Menge von 0,2 bis 5,0 Gew.-% und insbesondere bevorzugt in einer Menge von 0,5 bis 3,0 Gew.-% in den erfindungsgemäßen Zusammensetzungen enthalten.

Die gewünschte Viskosität der kosmetischen, dermatologischen oder pharmazeutischen Zusammensetzungen kann durch Zugabe von weiteren Verdickern und Gelierungsmittel eingestellt werden. In Betracht kommen vorzugsweise Celluloseether und andere Cellulosederivate (z. B. Carboxymethylcellulose, Hydroxyethylcellulose), Gelatine, Stärke und Stärkederivate, Natriumalginate, Fettsäurepolyethylenglykolester, Agar-Agar, Caragenan, Traganth oder Dextrinderivate, insbesondere Dextrinester. Des Weiteren eignen sich Metallsalze von Fettsäuren, bevorzugt mit 12 bis 22 C-Atomen, beispielsweise Natriumstearat, Natriumpalmitat, Natriumlaurat, Natriumarachidate, Natriumbehenat, Kaliumstearat, Kaliumpalmitat, Natriummyristat, Aluminiummonostearat, Hydroxyfettsäuren, beispielsweise 12-Hydroxystearinsäure, 16-Hydroxyhexadecanoylsäure; Fettsäureamide; Fettsäurealkanolamide; Dibenzalsorbit und alkohollösliche Polyamide und Polyacrylamide oder Mischungen solcher. Weiterhin können vernetzte und unvernetzte Polyacrylate wie Carbomer, Natriumpolyacrylate oder sulfonsäurehaltige Polymere wie Ammonium Acryloyldimethyltaurate/VP-Copolymer oder Sodium Acryloyldimethyltaurate/VP-Copolymer Verwendung finden.

Sofern die erfindungsgemäßen kosmetischen, dermatologischen oder pharmazeutischen Zusammensetzungen eine oder mehrere Substanzen ausgewählt aus der Gruppe bestehend aus den weiteren Verdickern und Geliermitteln enthalten, ist diese eine oder sind diese mehreren Substanzen, bezogen auf das Gesamtgewicht der entsprechenden erfindungsgemäßen Zusammensetzung, vorzugsweise in einer Menge von 0,01 bis 20,0 Gew.-%, besonders bevorzugt in einer Menge von 0,1 bis 10,0 Gew.-%, insbesondere bevorzugt in einer Menge von 0,2 bis 3,0 Gew.-% und außerordentlich bevorzugt in einer Menge von 0,4 bis 2,0 Gew.-% in den erfindungsgemäßen Zusammemnsetzungen enthalten.

Als Überfettungsmittel oder Rückfetter können vorzugsweise Lanolin und Lecithin, nicht ethoxylierte und polyethoxylierte oder acylierte Lanolin- und Lecithinderivate, Polyolfettsäureester wie Glyceryloleat, Mono-, Di- und Triglyceride und/oder Fettsäurealkanolamide, wobei die letzteren gleichzeitig als Schaumstabilisatoren dienen, verwendet werden. Sofern die erfindungsgemäßen kosmetischen, dermatologischen oder pharmazeutischen Zusammensetzungen eine oder mehrere der soeben genannten Substanzen enthalten, ist diese eine oder sind diese mehreren Substanzen, bezogen auf das Gesamtgewicht der entsprechenden erfindungsgemäßen Zusammensetzung, vorzugsweise in einer Menge von 0,01 bis 10,0 Gew.-%, besonders bevorzugt in einer Menge von 0,1 bis 5,0 Gew.-% und insbesondere bevorzugt in einer Menge von 0,5 bis 3,0 Gew.-% in den erfindungsgemäßen Zusammensetzungen enthalten.

In einer weiteren bevorzugten Ausführungsform der Erfindung enthalten die erfindungsgemäßen kosmetischen, dermatologischen oder pharmazeutischen Zusammensetzungen ein oder mehrere weitere antimikrobielle Wirkstoffe und liegen vorzugsweise in der Form von Desinfektionszusammensetzungen und besonders bevorzugt in der Form von Desinfektionsgelen vor.

An weiteren antimikrobiellen Wirkstoffen können Cetyltrimethylammoniumchlorid, Cetylpyridiniumchlorid, Benzethoniumchlorid, Diisobutylethoxyethyldimethylbenzylammoniumchlorid, Natrium N-Laurylsarcosinat, Natrium-N-Palmethylsarcosinat, Lauroylsarcosin, N-Myristoylglycin, Kalium-N-Laurylsarcosin, Trimethylammoniumchlorid, Natriumaluminiumchlorohydroxylactat, Triethylcitrat, Tricetylmethylammoniumchlorid, 2,4,4'-Trichloro-2'-hydroxydiphenylether (Triclosan), Phenoxyethanol, 1,5-Pentandiol, 1,6-Hexandiol, 3,4,4'-Trichlorocarbanilid (Triclocarban), Diaminoalkylamid, beispielsweise L-Lysinhexadecylamid, Citratschwermetallsalze, Salicylate, Piroctose, insbesondere Zinksalze, Pyrithione und deren Schwermetall salze, insbesondere Zinkpyrithion, Zinkphenolsulfat, Farnesol, Ketoconazol, Oxiconazol, Bifonazole, Butoconazole, Cloconazole, Clotrimazole, Econazole, Enilconazole, Fenticonazole, Isoconazole, Miconazole, Sulconazole, Tioconazole, Fluconazole, Itraconazole, Terconazole, Naftifine und Terbinafine, Selendisulfid und Octopirox®, Iodopropynylbutylcarbamat, Methylchloroisothiazolinon, Methylisothiazolinon, Methyldibromo Glutaronitril, AgCl, Chloroxylenol, Na-Salz von Diethylhexylsulfosuccinat, Natriumbenzoat, sowie Phenoxyethanol, Benzylalkohol, Phenoxyisopropanol, Parabene, bevorzugt Butyl-, Ethyl-, Methyl- und Propylparaben, sowie deren Na-Salze, 2-Bromo-2-Nitropropan-1,3-diol, Benzylalkohol, Sorbinsäure, Benzoesäure, Milchsäure, Imidazolidinylharnstoff, Diazolidinylharnstoff, Dimethyloldimethylhydantoin (DMDMH), Na-Salz von Hydroxymethylglycinat, Hydroxyethylglycin der Sorbinsäure und Kombinationen dieser Wirksubstanzen zum Einsatz kommen.

Sofern die erfindungsgemäßen kosmetischen, dermatologischen oder pharmazeutischen Zusammensetzungen einen oder mehrere weitere antimikrobielle Wirkstoffe enthalten, sind diese, bezogen auf das Gesamtgewicht der entsprechenden erfindungsgemäßen Zusammensetzung, vorzugsweise in einer Menge von 0,001 bis 5,0 Gew.-%, besonders bevorzugt in einer Menge von 0,01 bis 3,0 Gew.-% und insbesondere bevorzugt in einer Menge von 0,1 bis 2,0 Gew.-% in den erfindungsgemäßen Zusammensetzungen enthalten.

Die erfindungsgemäßen kosmetischen, dermatologischen oder pharmazeutischen Zusammensetzungen können des Weiteren biogene Wirkstoffe, ausgewählt aus Pflanzenextrakten, wie beispielsweise Aloe Vera, sowie Lokalanästhetika, Antibiotika, Antiphlogistika, Antiallergica, Corticosteroide, Sebostatika, Bisabolol®, Allantoin, Phytantriol®, Proteine, Vitamine ausgewählt aus Niacin, Biotin, Vitamin B2, Vitamin B3, Vitamin B6, Vitamin B3 Derivaten (Salzen,

Säuren, Estern, Amiden, Alkoholen), Vitamin C und Vitamin C Derivaten (Salzen, Säuren, Estern, Amiden, Alkoholen), bevorzugt als Natriumsalz des Monophosphorsäureesters der Ascorbinsäure oder als Magnesiumsalz des Phosphorsäureesters der Ascorbinsäure, Tocopherol und Tocopherolacetat, sowie Vitamin E und/oder dessen Derivate enthalten.

Sofern die erfindungsgemäßen kosmetischen, dermatologischen oder pharmazeutischen Zusammensetzungen einen oder mehrere biogene Wirkstoffe enthalten, sind diese, bezogen auf das Gesamtgewicht der entsprechenden erfindungsgemäßen Zusammensetzung, vorzugsweise in einer Menge von 0,001 bis 5,0 Gew.-%, besonders bevorzugt in einer Menge von 0,01 bis 3,0 Gew.-% und insbesondere bevorzugt in einer Menge von 0,1 bis 2,0 Gew.-% in den erfindungsgemäßen Zusammensetzungen enthalten.

Die erfindungsgemäßen kosmetischen, dermatologischen oder pharmazeutischen Zusammensetzungen können Adstringentien, bevorzugt Magnesiumoxid, Aluminiumoxid, Titandioxid, Zirkondioxid und Zinkoxid, Oxidhydrate, bevorzugt Aluminiumoxidhydrat (Böhmit) und Hydroxide, bevorzugt von Calcium, Magnesium, Aluminium, Titan, Zirkon oder Zink, sowie Aluminiumchlorohydrate enthalten. Sofern die erfindungsgemäßen kosmetischen, dermatologischen oder pharmazeutischen Zusammensetzungen ein oder mehrere Adstringentien enthalten, sind diese, bezogen auf das Gesamtgewicht der entsprechenden erfindungsgemäßen Zusammensetzung, vorzugsweise in einer Menge von 0,001 bis 50,0 Gew.-%, besonders bevorzugt in einer Menge von 0,01 bis 10,0 Gew.-% und insbesondere bevorzugt in einer Menge von 0,1 bis 10,0 Gew.-% in den erfindungsgemäßen Zusammensetzungen enthalten.

Als deodorierende Stoffe bevorzugt sind Allantoin und Bisabolol. Sofern die erfindungsgemäßen kosmetischen, dermatologischen oder pharmazeutischen Zusammensetzungen ein oder mehrere deodorierende Stoffe enthalten, sind diese, bezogen auf das Gesamtgewicht der entsprechenden erfindungsgemäßen Zusammensetzung, vorzugsweise in einer Menge von 0,0001 bis 10,0 Gew.-% in den erfindungsgemäßen Zusammensetzungen enthalten.

In einer weiteren bevorzugten Ausführungsform der Erfindung enthalten die erfindungsgemäßen kosmetischen, dermatologischen oder pharmazeutischen Zusammensetzungen ein oder mehrere Substanzen ausgewählt aus anorganischen und organischen UV-Filtern und liegen besonders bevorzugt in der Form von Sonnenschutzzusammensetzungen vor.

Die erfindungsgemäßen kosmetischen, dermatologischen oder pharmazeutischen Zusammensetzungen können als Pigmente/Mikropigmente sowie als anorganische Sonnenschutzfilter bzw. UV-Filter mikrofeines Titandioxid, Glimmer-Titanoxid, Eisenoxide, Glimmer-Eisenoxid, Zinkoxid, Siliciumoxide, Ultramarinblau oder Chromoxide enthalten.

Die organischen Sonnenschutzfilter bzw. UV-Filter sind vorzugsweise ausgewählt aus 4-Aminobenzoesäure, 3-(4'-Trimethylammonium)-benzyliden-boran-2-on-methylsulfat, Camphor Benzalkonium Methosulfat, 3,3,5-Trimethylcyclohexylsalicylat, 2-Hydroxy-4-methoxybenzophenon, 2-Phenylbenzimidazol-5-sulfonsäure und ihre Kalium-, Natrium- und Triethanolaminsalze, 3,3'-(1,4-Phenylendimethin)-bis-(7,7-dimethyl-2-oxobicyclo[2.2.1]-heptan-1-methansulfonsäure) und ihre Salze, 1-(4-tert.-Butylphenyl)-3-(4-methoxyphenyl)propan-1,3-dion, 3-(4'-Sulfo)-benzyliden-bornan-2-on und seine Salze, 2-Cyan-3,3-diphenyl-acrylsäure-(2-ethylhexylester), Polymere von N-[2(und 4)-(2-oxoborn-3-ylidenmethyl)benzyl]-acrylamid, 4-Methoxy-zimtsäure-2-ethylhexylester, ethoxyliertes Ethyl-4-amino-benzoat, 4-Methoxy-zimtsäure-isoamylester, 2,4,6-Tris-[p-(2-ethylhexyloxycarbonyl)anilino]-1,3,5-triazin, 2-(2H-benzotriazol-2-yl)-4-methyl-6-(2-methyl-3-(1,3,3,3-tetramethyl-1-(trimethylsilyloxy)-disiloxanyl)-propyl)phenol, 4,4'-[(6-[4-((1,1-dimethylethyl)-amino-carbonyl)phenylamino]-1,3,5-triazin-2,4-yl)diimino]bis-(benzoesäure-2-ethylhexylester), Benzophenon-3, Benzophenon-4 (Säure), 3-(4'-Methylbenzyliden)-D,L-Campher, 3-Benzyliden-Campher, Salicylsäure-2-ethylhexylester, 4-Dimethylaminobenzoesäure-2-ethylhexylester, Hydroxy-4-methoxy-benzophenon-5-sulfonsäure (Sulfisobenzonum) und das Natriumsalz, 4-Isopropylbenzylsalicylat, N,N,N-Trimethyl-4-(2-oxoborn-3-ylidenemethyl) anilium methyl sulphate, Homosalate (INN), Oxybenzone (INN), 2-Phenylbenzimidazole-5-sulfonsäure und ihre Natrium-, Kalium-, und Triethanolaminsalze, Octylmethoxyzimtsäure, Isopentyl-4-methoxyzimtsäure, Isoamyl-p-methoxyzimtsäure, 2,4,6-Trianilino-(p-carbo-2'-ethylhexyl-1'-oxy)-1,3,5-triazine (Octyl triazone) Phenol, 2-2(2H-benzotriazol-2-yl)-4-methyl-6-(2-methyl-3-(1,3,3,3-tetramethyl-1-(trimethylsilyl)oxy)-disiloxanyl)propyl (Drometrizole Trisiloxane) benzoesäure, 4,4-((6-(((1,1-dimethylethyl)amino)carbonyl)phenyl)amino)-1,3,5-triazine-2,4-diyl)diimino)bis,bis(2-ethylhexyl)ester) benzoesäure, 4,4-((6-(((1,1-dimethylethyl)amino)-carbonyl)phenyl)amino)-1,3,5-triazine-2,4-diyl)diimino)bis,bis(2-ethylhexyl)ester), 3-(4'-Methylbenzyliden)-D,L-campher (4-Methylbenzyliden Camphor), Benzyliden-camphor-sulfonsäure, Octocrylen, Polyacrylamidomethyl-Benzyliden-Camphor, 2-Ethylhexyl salicylat (Octyl Salicylat), 4-Dimethyl-aminobenzoesäureethyl-2-hexylester (octyl dimethyl PABA), PEG-25 PABA, 2-Hydroxy-4-methoxybenzo-phenone-5-sulfonsäure (Benzophenone-5) und das Na-Salz, 2,2'-Methylen-bis-6-(2H-benzotriazol-2yl)-4-(tetramethyl-butyl)-1,1,3,3-phenol, Natriumsalz von 2-2'-bis-(1,4-phenylen)1H-benzimidazole-4,6-disulfonsäure, (1,3,5)-Triazine-2,4-bis((4-(2-ethyl-hexyloxy)-2-hydroxy)-phenyl)-6-(4-methoxyphenyl), 2-Ethylhexyl-2-cyano-3,3-diphenyl-2-propenoat, Glyceryl octanoat, Di-p-methoxyzimtsäure, p-Amino-benzoesäure und deren Ester, 4-tert-Butyl-4'-methoxydibenzoylmethan, 4-(2-β-Glucopyranoxy)propoxy-2-hydroxybenzophenon, Octyl Salicylat, Methyl-2,5-diisopropylzimtsäure, Cinoxat, Dihydroxy-dimethoxybenzophenon, Dinatriumsalz von 2,2'-Dihydroxy-4,4'-dimethoxy-5,5'-disulfobenzophenon, Dihydroxybenzophenon, 1,3,4-Dimethoxyphenyl-4,4-dimethyl-1,3-pentanedion, 2-Ethylhexyl-dimethoxybenzyliden-dioxoimidazolidinpropionat, Methylen-Bis-Benztriazolyl Tetramethylbutylphenol, Phenyldibenzimidazoltetrasulfonat, Bis-Ethylhexyloxyphenol-Methoxyphenol-Triazin, Tetrahydroxybenzophenone, Terephthalylidendicampher-sulfonsäure, 2,4,6-tris[4,2-Ethylhexyloxycarbonyl)anilino]-1,3,5-triazin, Methyl-bis(trimethylsiloxy)silylisopentyl trimethoxy-zimtsäure, Amyl-p-dimethylaminobenzoat, Amyl-p-dimethylamino benzoat, 2-Ethylhexyl-p-dimethylaminobenzoat, Isopropyl-p-methoxyzimtsäure/ Diisopropylzimtsäureester, 2-Ethylhexyl-p-methoxyzimtsäure, 2-Hydroxy-4-methoxy benzophenon, 2-Hydroxy-4-methoxy-benzophenon-5-sulfsäure und das Trihydrat, sowie 2-Hydroxy-4-methoxybenzophenon-5-sulfonat Natriumsalz und Phenyl-benzimidazol-sulfonsäure.

Sofern die erfindungsgemäßen kosmetischen, dermatologischen oder pharmazeutischen Zusammensetzungen einen oder mehrere Sonnenschutzfilter enthalten, sind diese, bezogen auf das Gesamtgewicht der entsprechenden erfindungsgemäßen Zusammensetzung, vorzugsweise in einer Menge von 0,001 bis 30,0 Gew.-%, besonders bevorzugt in einer Menge von 0,05 bis 20,0 Gew.-% und insbesondere bevorzugt in einer Menge von 1,0 bis 10,0 Gew.-% in den erfindungsgemäßen Zusammensetzungen enthalten.

Die erfindungsgemäßen kosmetischen, dermatologischen oder pharmazeutischen Zusammensetzungen können ein oder mehrere Antioxidantien enthalten, vorzugsweise ausgewählt aus Aminosäuren (z. B. Glycin, Histidin, Tyrosin, Tryptophan) und deren Derivaten, Imidazolen (z. B. Urocaninsäure) und deren Derivaten, Peptiden wie D,L-Carnosin, D-Carnosin, L-Carnosin und deren Derivaten (z. B. Anserin), Carotinoiden, Carotinen (z. B. $\alpha$-Carotin, $\beta$-Carotin, Lycopin) und deren Derivaten, Chlorogensäure und deren Derivaten, Liponsäure und deren Derivaten (z. B. Dihydroliponsäure), Aurothioglucose, Propylthiouracil und anderen Thiolen (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, $\gamma$-Linoleyl-, Cholesteryl- und Glycerylester) sowie deren Salzen, Dilaurylthiodipropionat, Distearylthiodipropionat, Thiodipropionsäure und deren Derivaten (z. B. Ester, Ether, Peptide, Lipide, Nukleotide, Nukleoside und Salze), sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen, ferner (Metall)-Chelatoren (z. B. $\alpha$-Hydroxyfettsäuren, Palmitinsäure, Phytinsäure, Lactoferrin), $\alpha$-Hydroxysäuren (z. B. Citronensäure, Milchsäure, Äpfelsäure), Huminsäure, Gallensäure, Gallenextrakten, Bilirubin, Biliverdin, EDTA, EGTA und deren Derivaten, ungesättigten Fettsäuren und deren Derivaten (z. B. $\gamma$-Linotensäure, Linolsäure, Ölsäure), Folsäure und deren Derivaten, Ubichinon und Ubichinol und deren Derivaten, Vitamin C und Derivaten (z. B. Ascorbylpalmitat, Mg-Ascorbylphosphat, Ascorbylacetat), Tocopherolen und Derivaten (z. B. Vitamin E-acetat), Vitamin A und Derivaten (Vitamin A-palmitat) sowie Koniferylbenzoat des Benzoeharzes, Rutinsäure und deren Derivaten, $\alpha$-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivaten, Mannose und deren Derivaten, Zink und dessen Derivaten (z. B. ZnO, $ZnSO_4$), Selen und dessen Derivaten (z. B. Selenmethionin), Stilbenen und deren Derivaten (z. B. Stilbenoxid, trans-Stilbenoxid), Superoxid-Dismutase und den erfindungsgemäß geeigneten Derivaten (Salzen, Estern, Ethern, Zuckern, Nukleotiden, Nukleosiden, Peptiden und Lipiden) dieser genannten Stoffe.

Die Antioxidantien können die Haut und das Haar vor oxidativer Beanspruchung schützen. Bevorzugte Antioxidantien sind dabei Vitamin E und dessen Derivate sowie Vitamin A und dessen Derivate.

Sofern die erfindungsgemäßen kosmetischen, dermatologischen oder pharmazeutischen Zusammensetzungen einen oder mehrere Antioxidantien enthalten, sind diese, bezogen auf das Gesamtgewicht der entsprechenden erfindungsgemäßen Zusammensetzung, vorzugsweise in einer Menge von 0,001 bis 30,0 Gew.-%, besonders bevorzugt in einer Menge von 0,05 bis 20,0 Gew.-% und insbesondere bevorzugt in einer Menge von 1,0 bis 10,0 Gew.-% in den erfindungsgemäßen Zusammensetzungen enthalten.

Des Weiteren können Feuchthaltemittel, ausgewählt aus dem Natriumsalz von 2-Pyrrolidone-5-carboxylat (NaPCA), Guanidin; Glycolsäure und deren Salzen, Milchsäure und deren Salzen, Glucosamine und deren Salzen, Lactamidmonoethanolamin, Acetamidmonoethanolamin, Harnstoff, Hydroxyethylharnstoff, Hydroxysäuren, Panthenol und dessen Derivaten, beispielsweise D-Panthenol (R-2,4-Dihydroxy-N-(3-hydroxypropyl)-3,3-dimethylbutamid), D,L-Panthenol, Calciumpantothenat, Panthetin, Pantothein, Panthenylethylether, Isopropylpalmitat und/oder Glycerin eingesetzt werden. Sofern die erfindungsgemäßen Zusammensetzungen ein oder mehrere Feuchthaltemittel enthalten, sind diese, bezogen auf das Gesamtgewicht der entsprechenden erfindungsgemäßen Zusammensetzung, vorzugsweise in einer Menge von 0,1 bis 15,0 Gew.-% und besonders bevorzugt in einer Menge von 0,5 bis 5,0 Gew.-% in den erfindungsgemäßen Zusammensetzungen enthalten.

Zusätzlich können die erfindungsgemäßen kosmetischen, dermatologischen oder pharmazeutischen Zusammensetzungen organische Lösungsmittel enthalten. Prinzipiell kommen als organische Lösungsmittel alle ein- oder mehrwertigen Alkohole in Betracht. Bevorzugt werden Alkohole mit 1 bis 4 Kohlenstoffatomen wie Ethanol, Propanol, Isopropanol, n-Butanol, i-Butanol, tert.-Butanol, Glycerin und Mischungen aus den genannten Alkoholen eingesetzt. Weitere bevorzugte Alkohole sind Polyethylenglykole mit einer relativen Molekülmasse unter 2000. Insbesondere ist ein Einsatz von Polyethylenglykol mit einer relativen Molekülmasse zwischen 200 und 600 und in Mengen bis zu 45,0 Gew.-% und von Polyethylenglykol mit einer relativen Molekülmasse zwischen 400 und 600 in Mengen von 5,0 bis 25,0 Gew.-% bevorzugt. Weitere geeignete Lösungsmittel sind beispielsweise Triacetin (Glycerintriacetat) und 1-Methoxy-2-propanol.

Die erfindungsgemäßen kosmetischen, dermatologischen oder pharmazeutischen Zusammensetzungen können ein oder mehrere Substanzen ausgewählt aus Farbmitteln, z. B. Farbstoffe und/oder Pigmente, enthalten. Die in den erfindungsgemäßen kosmetischen, dermatologischen oder pharmazeutischen Zusammensetzungen enthaltenen Farbstoffe und/oder Pigmente, sowohl organische als auch anorganische Farbstoffe und Pigmente, sind aus der entsprechenden Positivliste der Kosmetikverordnung bzw. der EG-Liste kosmetischer Färbemittel ausgewählt.

| Chemische oder sonstige Bezeichnung | CIN | Farbe |
|---|---|---|
| Pigment Green | 10006 | Grün |
| Acid Green 1 | 10020 | Grün |
| 2,4-Dinitrohydroxynaphtalin-7-sulfosäure | 10316 | Gelb |
| Pigment Yellow 1 | 11680 | Gelb |
| Pigment Yellow 3 | 11710 | Gelb |
| Pigment Orange 1 | 11725 | Orange |
| 2,4-Dihydroxyazobenzol | 11920 | Orange |
| Solvent Red 3 | 12010 | Rot |
| 1-(2'-Chlor-4'-nitro-1'-phenylazo)-2-hydroxynaphthalin | 12085 | Rot |
| Pigment Red 3 | 12120 | Rot |
| Ceresrot; Sudanrot; Fettrot G | 12150 | Rot |
| Pigment Red 112 | 12370 | Rot |
| Pigment Red 7 | 12420 | Rot |
| Pigment Brown 1 | 12480 | Braun |
| 4-(2'-Methoxy-5'-sulfosäurediethylamid-1'-phenylazo)-3-hydroxy-5"-chloro-2",4"-dimethoxy-2-naphthoesäureanilid | 12490 | Rot |
| Disperse Yellow 16 | 12700 | Gelb |
| 1-(4-Sulfo-1-phenylazo)-4-amino-brezol-sulfosäure | 13015 | Gelb |
| 2,4-Dihydroxy-azobenzol-4'-sulfosäure | 14270 | Orange |
| 2-(2,4-Dimethylphenylazo-5-sulfosäurse)-1-hydroxynaphthalin-4-sulfosäure | 14700 | Rot |
| 2-(4-Sulfo-1-naphthylazo)-1-naphthol-4-sulfosäure | 14720 | Rot |
| 2-(6-Sulfo-2,4-xylylazo)-1-naphthol-5-sulfosäure | 14815 | Rot |
| 1-(4'-Sulfophenylazo)-2-hydroxynaphthalin | 15510 | Orange |
| 1-(2-Sulfosäure-4-chlor-5-carbonsäure-1-phenylazo)-2-hydroxynaphthalin | 15525 | Rot |
| 1-(3-Methyl-phenylazo-4-sulfosäure)-2-hydroxynaphthalin | 15580 | Rot |
| 1-(4',(8')-Sulfosäurenaphthylazo)-2-hydroxynaphthalin | 15620 | Rot |
| 2-Hydroxy-1,2'-azonaphthalin-1'-sulfosäure | 15630 | Rot |
| 3-Hydroxy-4-phenylazo-2-naphthylcarbonsäure | 15800 | Rot |
| 1-(2-Sulfo-4-methyl-1-phenylazo)-2-naphthylcarbonsäure | 15850 | Rot |
| 1-(2-Sulfo-4-methyl-5-chlor-1-phenylazo)-2-hydroxynaphthalin-3-carbonsäure | 15865 | Rot |
| 1-(2-Sulfo-1-naphthylazo)-2-hydroxynaphthalin-3-carbonsäure | 15880 | Rot |
| 1-(3-Sulfo-1-phenylazo)-2-naphthol-6-sulfosäure | 15980 | Orange |
| 1-(4-Sulfo-1-phenylazo)-2-naphthol-6-sulfosäure | 15985 | Gelb |
| Allura Red | 16035 | Rot |
| 1-(4-Sulfo-1-naphthylazo)-2-naphthol-3,6-disulfosäure | 16185 | Rot |
| Acid Orange 10 | 16230 | Orange |

(fortgesetzt)

| Chemische oder sonstige Bezeichnung | CIN | Farbe |
|---|---|---|
| 1-(4-Sulfo-1-naphthylazo)-2-naphthol-6,8-disulfosäure | 16255 | Rot |
| 1-(4-Sulfo-1-naphthylazo)-2-naphthol-3,6,8,-trisulfosäure | 16290 | Rot |
| 8-Amino-2-phenylazo-1-naphthol-3,6-disulfosäure | 17200 | Rot |
| Acid Red 1 | 18050 | Rot |
| Acid Red 155 | 18130 | Rot |
| Acid Yellow 121 | 18690 | Gelb |
| Acid Red 180 | 18736 | Rot |
| Acid Yellow 11 | 18820 | Gelb |
| Acid Yellow 17 | 18965 | Gelb |
| 4-(4-Sulfo-1-phenylazo)-1-(4-sulfophenyl)-5-hydroxyphrazolon-3-carbonsäure | 19140 | Gelb |
| Pigment Yellow 16 | 20040 | Gelb |
| 2,6-(4'-Sulfo-2",4"-dimethyl)-bis-phenylazo)1,3-dihydroxybenzol | 20170 | Orange |
| Acid Black 1 | 20470 | Schwarz |
| Pigment Yellow 13 | 21100 | Gelb |
| Pigment Yellow 83 | 21108 | Gelb |
| Solvent Yellow | 21230 | Gelb |
| Acid Red 163 | 24790 | Rot |
| Acid Red 73 | 27290 | Rot |
| 2-[4'-(4"Sulfo-1"-phenylazo)-7'-sulfo-1'-naphthylazo]-1-hydroxy-7-aminoaphthalin-3,6-disulfosäure | 27755 | Schwarz |
| 4'-[(4"-Sulfo-1"-phenylazo)-7'-sulfo-1'-naphthylazo]-1-hydroxy-8-acetyl-aminonaphthalin-3,5-disulfosäure | 28440 | Schwarz |
| Direct Orange 34, 39, 44, 46, 60 | 40215 | Orange |
| Food Yellow | 40800 | Orange |
| trans-ß-Apo-8'-Carotinaldehyd ($C_{30}$) | 40820 | Orange |
| trans-Apo-8'-Carotinsäure ($C_{30}$)-ethylester | 40825 | Orange |
| Canthaxanthin | 40850 | Orange |
| Acid Blue 1 | 42045 | Blau |
| 2,4-Disulfo-5-hydroxy-4'-4"-bis-(diethylamino)triphenylcarbinol | 42051 | Blau |
| 4-[(-4-N-Ethyl-p-sulfobenzylamino)-phenyl-(4-hydroxy-2-sulfophenyl)-(methylen)-1-(N-ethylN-p-sulfobenzyl)-2,5-cyclohexadienimin] | 42053 | Grün |
| Acid Blue 7 | 42080 | Blau |
| (N-Ethyl-p-sulfobenzyl-amino-phenyl-(2-sulfophenyl)-methylen-(N-ethyl-N-p-sulfo-benzyl)-cyclohexadienimin | 42090 | Blau |
| Acid Green 9 | 42100 | Grün |
| Diethyl-di-sulfobenzyl-di-4-amino-2-chlor-di-2-methyl-fuchsonimonium | 42170 | Grün |
| Basic Violet 14 | 42510 | Violett |
| Basic Violet 2 | 42520 | Violett |

(fortgesetzt)

| Chemische oder sonstige Bezeichnung | CIN | Farbe |
|---|---|---|
| 2'-Methyl-4'-(N-ethyl-N-m-sulfobenzyl)-amino-4"-(N-diethyl)-amino-2-methyl-N-ethyl-N-m-sulfobenzyl-fuchsonimmonium | 42735 | Blau |
| 4'-(N-Dimethyl)-amino-4"-(N-phenyl)-aminonaphtho-N-dimethyl-fuchsonimmonium | 44045 | Blau |
| 2-Hydroxy-3,6-disulfo-4,4'-bis-dimethylaminonaphtofuchsinimmonium | 44090 | Grün |
| Acid red | 45100 | Rot |
| 3-(2'-Methylphenylamino)-6-(2'-methyl-4'-sulfophenylamino)-9-(2"-carboxyphenyl)-xantheniumsalz | 45190 | Violett |
| Acid Red 50 | 45220 | Rot |
| Phenyl-2-oxyfluoron-2-carbonsäure | 45350 | Gelb |
| 4,5-Dibromfluorescein | 45370 | Orange |
| 2,4,5,7-Tetrabromfluorescein | 45380 | Rot |
| Solvent Dye | 45396 | Orange |
| Acid Red 98 | 45405 | Rot |
| 3',4',5',6'-Tetrachlor-2,4,5,7-tetrabromfluorescein | 45410 | Rot |
| 4,5-Diiodfluorescein | 45425 | Rot |
| 2,4,5,7-Tetraiodfluorescein | 45430 | Rot |
| Chinophthalon | 47000 | Gelb |
| Chinophthalon-disulfosäure | 47005 | Gelb |
| Acid violet 50 | 50325 | Violett |
| Acid Black 2 | 50420 | Schwarz |
| Pigment Violet 23 | 51319 | Violett |
| 1,2-Dioxyanthrachinon, Calcium-Aluminiumkomplex | 58000 | Rot |
| 3-Oxypyren-5,8,10-sulfosäure | 59040 | Grün |
| 1-Hydroxy-4-N-phenyl-aminoanthrachinon | 60724 | Violett |
| 1-Hydroxy-4-(4'-methylphenylamino)-anthrachinon | 60725 | Violett |
| Acid Violet 23 | 60730 | Violett |
| 1,4-Di(4'-methyl-phenylamino)-anthrachinon | 61565 | Grün |
| 1,4-Bis-(o-sulfo-p-toluidine)-anthrachinon | 61570 | Grün |
| Acid Blue 80 | 61585 | Blau |
| Acid Blue 62 | 62045 | Blau |
| N,N'-Dihydro-1,2,1',2'-anthrachinonazin | 69800 | Blau |
| Vat Blue 6; Pigment Blue 64 | 69825 | Blau |
| Vat Orange 7 | 71105 | Orange |
| Indigo | 73000 | Blau |
| Indigo-disulfosäure | 73015 | Blau |
| 4,4'-Dimethyl-6,6'-dichlorthioindigo | 73360 | Rot |
| 5,5'-Dichlor-7,7'-dimethylthioindigo | 73385 | Violett |
| Quinacridone Violet 19 | 73900 | Violett |

(fortgesetzt)

| Chemische oder sonstige Bezeichnung | CIN | Farbe |
|---|---|---|
| Pigment Red 122 | 73915 | Rot |
| Pigment Blue 16 | 74100 | Blau |
| Phthalocyanine | 74160 | Blau |
| Direct Blue 86 | 74180 | Blau |
| chlorierte Phthalocyanine | 74260 | Grün |
| Natural Yellow 6,19; Natural Red 1 | 75100 | Gelb |
| Bixin, Nor-Bixin | 75120 | Orange |
| Lycopin | 75125 | Gelb |
| trans-alpha, beta- bzw. gamma-Carotin | 75130 | Orange |
| Keto- und/oder Hydroxylderivate des Carotins | 75135 | Gelb |
| Guanin oder Perlglanzmittel | 75170 | Weiß |
| 1,7-Bis-(4-hydroxy-3-methoxyphenyl)1,6-heptadien-3,5-dion | 75300 | Gelb |
| Komplexsalz (Na,Al,Ca) der Karminsäure | 75470 | Rot |
| Chlorophyll a und b; Kupferverbindungen der Chlorophylle und Chlorophylline | 75810 | Grün |
| Aluminium | 77000 | Weiß |
| Tonerdehydrat | 77002 | Weiß |
| wasserhaltige Aluminiumsilikate | 77004 | Weiß |
| Ultramarin | 77007 | Blau |
| Pigment Red 101 und 102 | 77015 | Rot |
| Bariumsulfat | 77120 | Weiß |
| Bismutoxychlorid und seine Gemische mit Glimmer | 77163 | Weiß |
| Calciumcarbonat | 77220 | Weiß |
| Calciumsulfat | 77231 | Weiß |
| Kohlenstoff | 77266 | Schwarz |
| Pigment Black 9 | 77267 | Schwarz |
| Carbo medicinalis vegetabilis | 77268:1 | Schwarz |
| Chromoxid | 77288 | Grün |
| Chromoxid, wasserhaltig | 77289 | Grün |
| Pigment Blue 28, Pigment Green 14 | 77346 | Grün |
| Pigment Metal 2 | 77400 | Braun |
| Gold | 77480 | Braun |
| Eisenoxide und -hydroxide | 77489 | Orange |
| Eisenoxide und -hydroxide | 77491 | Rot |
| Eisenoxidhydrat | 77492 | Gelb |
| Eisenoxid | 77499 | Schwarz |
| Mischungen aus Eisen(II)- und Eisen(III)-hexacyanoferrat | 77510 | Blau |
| Pigment White 18 | 77713 | Weiß |
| Mangananimoniumdiphosphat | 77742 | Violett |

(fortgesetzt)

| Chemische oder sonstige Bezeichnung | CIN | Farbe |
|---|---|---|
| Manganphosphat; $Mn_3(PO_4)_2*7H_2O$ | 77745 | Rot |
| Silber | 77820 | Weiß |
| Titandioxid und seine Gemische mit Glimmer | 77891 | Weiß |
| Zinkoxid | 77947 | Weiß |
| 6,7-Dimethyl-9-(1 -D-ribityl)-isoalloxazin, Lactoflavin | | Gelb |
| Zuckerkulör | | Braun |
| Capsanthin, Capsorubin | | Orange |
| Betanin | | Rot |
| Benzopyriliumsalze, Anthocyanine | | Rot |
| Aluminium-, Zink-, Magnesium-, und Calciumstearat | | Weiß |
| Bromthymolblau | | Blau |
| Bromkresolgrün | | Grün |
| Acid Red 195 | | Rot |

Ferner vorteilhaft sind öllösliche Naturfarbstoffe, wie z. B. Paprikaextrakte, β-Carotin und Cochenille.

Vorteilhaft eingesetzt werden auch Perlglanzpigmente, z. B. Fischsilber (Guanin/ Hypoxanthin-Mischkristalle aus Fischschuppen) und Perlmutt (vermahlene Muschelschalen), monokristalline Perlglanzpigmente wie z. B. Bismuthoxychlorid (BiOCl), Schicht-Substrat Pigmente, z. B. Glimmer/Metalloxid, silberweiße Perlglanzpigmente aus $TiO_2$, Interferenpigmente ($TiO_2$, unterschiedliche Schichtdicke), Farbglanzpigmente ($Fe_2O_3$) und Kombinationspigmente ($TiO_2/Fe_2O_3$, $TiO_2/Cr_2O_3$, $TiO_2$/Berliner Blau, $TiO_2$/Carmin).

Unter Effektpigmenten sind im Rahmen der vorliegenden Erfindung Pigmente zu verstehen, die durch ihre Brechungseigenschaften besondere optische Effekte hervorrufen. Effektpigmente verleihen der behandelten Oberfläche (Haut, Haar, Schleimhaut) Glanz- oder Glittereffekte oder können durch diffuse Lichtstreuung Hautunebenheiten und Hautfältchen optisch kaschieren. Als besondere Ausführungsform der Effektpigmente sind Interferenzpigmente bevorzugt. Besonders geeignete Effektpigmente sind beispielsweise Glimmerpartikel, die mit mindestens einem Metalloxid beschichtet sind. Neben Glimmer, einem Schichtsilikat, sind auch Kieselgel und andere $SiO_2$-Modifikationen als Träger geeignet. Ein häufig zur Beschichtung verwendetes Metalloxid ist beispielsweise Titanoxid, dem gewünschtenfalls Eisenoxid beigemischt sein kann. Über die Größe und die Form (z. B. sphärisch, ellipsoid, abgeflacht, eben, uneben) der Pigmentpartikel sowie über die Dicke der Oxidbeschichtung können die Reflexionseigenschaften beeinflusst werden. Auch andere Metalloxide, z. B. Bismutoxychlorid (BiOCl), sowie die Oxide von beispielsweise Titan, insbesondere die $TiO_2$-Modifikationen Anatas und Rutil, Aluminium, Tantal, Niob, Zirkon und Hafnium. Auch mit Magnesiumfluorid ($MgF_2$) und Calciumfluorid (Flussspat, $CaF_2$) können Effektpigmente hergestellt werden.

Die Effekte lassen sich sowohl über die Partikelgröße als auch über die Partikelgrößenverteilung des Pigmentensembles steuern. Geeignete Partikelgrößenverteilungen reichen z. B. von 2 - 50 μm, 5 - 25 μm, 5 - 40 μm, 5 - 60 μm, 5 - 95 μm, 5 - 100 μm, 10 - 60 μm, 10 - 100 μm, 10 - 125 μm, 20 - 100 μm, 20 - 150 μm, sowie < 15 μm. Eine breitere Teilchengrößenverteilung z. B. von 20 - 150 μm, ruft glitzernde Effekte hervor, während eine engere Teilchengrößenverteilung von < 15 pm für eine gleichmäßige seidige Erscheinung sorgt.

Sofern die erfindungsgemäßen kosmetischen, dermatologischen oder pharmazeutischen Zusammensetzungen ein oder mehrere Effektpigmente enthalten, sind diese, bezogen auf das Gesamtgewicht der entsprechenden erfindungsgemäßen Zusammensetzung, vorzugsweise in einer Menge von 0,1 bis 20,0 Gew.-%, besonders bevorzugt in einer Menge von 0,5 bis 10,0 Gew.-% und insbesondere bevorzugt in einer Menge von 1,0 bis 5,0 Gew.-% in den erfindungsgemäßen Zusammensetzungen enthalten.

Als perlglanzgebende Komponente bevorzugt geeignet sind Fettsäuremonoalkanolamide, Fettsäuredialkanolamide, Monoester oder Diester von Alkylenglykolen, insbesondere Ethylenglykol und/oder Propylenglykol oder dessen Oligomere, mit höheren Fettsäuren, wie z. B. Palmitinsäure, Stearinsäure und Behensäure, Monoester oder Polyester von Glycerin mit Carbonsäuren, Fettsäuren und deren Metallsalze (im Falle der Monoester mit Carbonsäuren mit 14 bis 32 Kohlenstoffatomen), Ketosulfone oder Gemische der genannten Verbindungen.

Besonders bevorzugt sind Ethylenglykoldistearate und/oder Polyethylenglykoldistearate mit durchschnittlich 3 Glykoleinheiten.

Sofern die erfindungsgemäßen kosmetischen, dermatologischen oder pharmazeutischen Zusammensetzungen eine oder mehrere perlglanzgebende Verbindungen enthalten, sind diese, bezogen auf das Gesamtgewicht der entsprechenden erfindungsgemäßen Zusammensetzung, vorzugsweise in einer Menge von 0,1 bis 15,0 Gew.-% und besonders bevorzugt in einer Menge von 1,0 bis 10,0 Gew.-% in den erfindungsgemäßen Zusammensetzungen enthalten.

Als Duft- bzw. Parfümöle können einzelne Riechstoffverbindungen, z. B. die synthetischen Produkte vom Typ der Ester, Ether, Aldehyde, Ketone, Alkohole und Kohlenwasserstoffe verwendet werden. Riechstoffverbindungen vom Typ der Ester sind z. B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z. B. die linearen Alkanale mit 8 bis 18 C-Atomen, Citral, Citronellal, Citronellyloxyacetaldehyd, Cycllamenaldehyd, Hydroxycitronellal, Lilial und Bourgeonal, zu den Ketonen z. B. die lonone, alpha-Isomethylionon und Methyl-cedrylketon, zu den Alkoholen Anethol, Citronellol, Eugenol, Geranion, Linalol, Phenylethylalkohol und Terpineol, zu den Kohlenwasserstoffen gehören hauptsächlich die Terpene und Balsame. Bevorzugt werden Mischungen verschiedener Riechstoffe verwendet, die gemeinsam eine ansprechende Duftnote erzeugen.

Parfümöle können auch natürliche Riechstoffgemische enthalten, wie sie aus pflanzlichen oder tierischen Quellen zugänglich sind, z. B. Pinien-, Citrus-, Jasmin-, Lilien-, Rosen-, oder Ylang-Ylang-Öl. Auch ätherische Öle geringerer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl und Ladanumöl.

Als Trübungsmittel können Polymerdispersionen insbesondere Polyacrylatderivat-, Polyacrylamidderivat-, Poly(acrylatderivat-co-acrylamidderivat)-Dispersionen, Poly(styrolderivate-co-acrylatderivat)-Dispersionen, gesättigte und ungesättigte Fettalkohole verwendet werden.

An Silikonen können die zuvor unter den Silikonölen bzw. -wachsen genannten Substanzen verwendet werden.

Als Säuren oder Laugen zur pH-Wert Einstellung können vorzugsweise Mineralsäuren, insbesondere HCl, anorganische Basen, insbesondere NaOH oder KOH, und organische Säuren, insbesondere Zitronensäure, verwendet werden.

Die erfindungsgemäßen kosmetischen, dermatologischen oder pharmazeutischen Zusammensetzungen besitzen pH-Werte von vorzugsweise 2 bis 11, besonders bevorzugt von 4,5 bis 8,5 und insbesondere bevorzugt von 5,5 bis 8.

In einer weiteren bevorzugten Ausführungsform der Erfindung handelt es sich bei den erfindungsgemäßen Zusammensetzungen um Pflanzenschutzformulierungen. Diese Pflanzenschutzformulierungen werden im Folgenden beschrieben.

Die erfindungsgemäßen Pflanzenschutzformulierungen enthalten ein oder mehrere Pestizide.

Die erfindungsgemäßen Pflanzenschutzformulierungen enthalten die eine oder die mehreren Verbindungen der Komponente a) vorzugsweise in Mengen von 0,01 bis 10,0 Gew.-%, besonders bevorzugt in Mengen von 0,1 bis 5,0 Gew.-%, insbesondere bevorzugt in Mengen von 0,2 bis 3,0 Gew.-% und außerordentlich bevorzugt in Mengen von 0,5 bis 2,0 Gew.-% und die eine oder die mehreren Substanzen der Komponente b) vorzugsweise in Mengen von 0,01 bis 10,0 Gew.-%, besonders bevorzugt in Mengen von 0,1 bis 5,0 Gew.-%, insbesondere bevorzugt in Mengen von 0,2 bis 3,0 Gew.-% und außerordentlich bevorzugt in Mengen von 0,5 bis 2,0 Gew.-%, jeweils bezogen auf das Gesamtgewicht der erfindungsgemäßen Zusammensetzung.

Wie bereits erwähnt enthalten die erfindungsgemäßen Pflanzenschutzformulierungen in einer bevorzugten Ausführungsform der Erfindung keine Verbindungen ausgewählt aus Sorbitol und Sorbitolestern. Sofern die erfindungsgemäßen Pflanzenschutzformulierungen jedoch eine oder mehrere Verbindungen ausgewählt aus Sorbitol und Sorbitolestern (wobei die der Säurekomponente dieser Ester zu Grunde liegende Carbonsäure vorzugsweise Caprylsäure ist) enthalten, sind diese Verbindungen gemeinsam vorzugsweise in einer Menge kleiner oder gleich 0,1 Gew.-%, besonders bevorzugt in einer Menge kleiner oder gleich 0,06 Gew.-%, insbesondere bevorzugt in einer Menge kleiner oder gleich 0,02 Gew.-% und außerordentlich bevorzugt in einer Menge kleiner oder gleich 0,01 Gew.-% in den erfindungsgemäßen Pflanzenschutzformulierungen enthalten, wobei die Angaben in Gew.-% jeweils auf das Gesamtgewicht der fertigen erfindungsgemäßen Zusammensetzung bezogen sind.

Wie bereits erwähnt enthalten die erfindungsgemäßen Pflanzenschutzformulierungen in einer weiteren bevorzugten Ausführungsform der Erfindung keine Verbindungen ausgewählt aus Sorbitan und Sorbitanestern. Sofern die erfindungsgemäßen Pflanzenschutzformulierungen jedoch eine oder mehrere Verbindungen ausgewählt aus Sorbitan und Sorbitanestern (wobei die der Säurekomponente dieser Ester zu Grunde liegende Carbonsäure vorzugsweise Caprylsäure ist) enthalten, sind diese Verbindungen gemeinsam vorzugsweise in einer Menge kleiner oder gleich 0,4 Gew.-%, besonders bevorzugt in einer Menge kleiner oder gleich 0,2 Gew.-%, insbesondere bevorzugt in einer Menge kleiner oder gleich 0,1 Gew.-% und außerordentlich bevorzugt in einer Menge kleiner oder gleich 0,02 Gew.-% in den erfindungsgemäßen Pflanzenschutzformulierungen enthalten, wobei die Angaben in Gew.-% jeweils auf das Gesamtgewicht der fertigen erfindungsgemäßen Zusammensetzung bezogen sind.

In einer weiteren bevorzugten Ausführungsform der Erfindung haben die erfindungsgemäßen Pflanzenschutzformulierungen Viskositäten vorzugsweise im Bereich von 50 bis 200 000 mPa · s, besonders bevorzugt im Bereich von 500 bis 100 000 mPa · s, insbesondere bevorzugt im Bereich von 2 000 bis 50 000 mPa · s und außerordentlich bevorzugt im Bereich von 5 000 bis 30 000 mPa · s (20 °C, Brookfield RVT, RV-Spindel-Satz bei 20 Umdrehungen pro Minute).

Die erfindungsgemäßen Pflanzenschutzformulierungen sind vorzugsweise auf wässriger oder wässrig-alkoholischer Basis aufgebaut oder liegen als Lösungen, Emulsionen oder Dispersionen vor. Besonders bevorzugt liegen sie als Emulsionen vor und insbesondere bevorzugt liegen sie als Öl-in-Wasser Emulsionen vor.

Die erfindungsgemäßen Pflanzenschutzformulierungen besitzen pH-Werte von vorzugsweise 2 bis 11, besonders bevorzugt von 4,5 bis 8,5 und insbesondere bevorzugt von 5,5 bis 8.

In einer weiteren bevorzugten Ausführungsform der Erfindung handelt es sich bei den erfindungsgemäßen Zusammensetzungen um Wasch- oder Reinigungsmittel. Diese Wasch- und Reinigungsmittel werden im Folgenden beschrieben.

Bei den erfindungsgemäßen Wasch- und Reinigungsmitteln handelt es sich vorzugsweise um flüssige Wasch- und Reinigungsmittel wie z.B. Handgeschirrspülmittel, Oberflächenreiniger, Flüssigwaschmittel für Textilien, Weichspüler für Textilien, Klarspüler für Geschirrspülmaschinen und flüssige Reiniger für das automatische Geschirrspülen.

Die erfindungsgemäßen Wasch- und Reinigungsmittel enthalten neben Wasser nichtionische, anionische, kationischen oder amphotere Tenside (oberflächenaktive Substanzen) oder Mischungen dieser.

Als nichtionische oberflächenaktive Verbindungen kommen vorzugsweise in Betracht:

Anlagerungsprodukte von 1 bis 30 Mol Ethylenoxid und/oder 1 bis 10 Mol Propylenoxid an lineare Fettalkohole mit 8 bis 22 C-Atomen, an Fettsäuren mit 12 bis 22 C-Atomen, in Fettsäureester, insbesondere in Methylester, mit 12-22 C-Atomen, durch Insertion zwischen Carboxyl- und Alkylgruppe, an Alkylphenole mit 8 bis 15 C-Atomen in der Alkylgruppe und an Sorbitan- bzw. Sorbitolester. Ebenfalls vorzugsweise geeignet sind ethoxylierte Fettamine, Fettsäureamide und Fettsäurealkanolamide sowie deren Ethoxylate, Alkylpolyglykoside und Gemische von Verbindungen aus mehreren dieser Substanzklasse.

Als anionische Tenside können linare Alkylbenzolsulfonate, Alkansulfonate, Alkylsulfate, Ethersulfate und Ethercarbonsären mit 1-20 Einheiten Ethylenoxid, sowie Fettseifen Verwendung finden.

Als kationische Verbindungen kommen quaternäre Ammoniumverbindungen, insbesonder Dimethylalkylaminquat, Methyldialkylaminquats und Esterquats , insbesondere Triethanolaminesterquats zum Einsatz.

An amphoteren Tensiden stehen vorzugsweise zur Verfügung Alkylaminoalkylcarbonsäuren, Dimethylfettaminoxide, Amidoaminoxide, Betaine, Sulfobetaine und Imidazolinderivate.

Die erfindungsgemäßen Wasch- und Reinigungsmittel können weiterhin Lösungsmittel- und Lösungsvermittler wie Alkohole, inbesondere Ethanol, Isopropanol, Propanol, Isobutanol, Ethylenglykol und höhere Polyglykole, Propylenglykol, Glykolether, inbesondere Butylglykol und Butyldiglykol enthalten.

Zur Einstellung des pH-Wertes der erfindungsgemäßen Wasch- und Reinigungsmittel kommen Neutralisationsmittel wie Alkali- und Erdalkalihydroxide, z. B. Natriumhydroxid, Kaliumhydroxid, und/oder Alkanolamine, z. B. Monoethanolamin, Triethanolamin oder Diglykolamin oder saure Verbindungen, z. B. organische Säuren wie Milchsäure, Ameisensäure, Essigsäure oder Zitronensäure zum Einsatz.

Als weitere Einsatzstoffe der erfindungsgemäßen Wasch- und Reinigungsmittel können Additive zum Einstellen der Viskosität, z. B. Verdicker, Komplexbildner gegen Wasserhärte, anorganische Builder wie Phosphate, Silikate, Carbonate oder organische Gerüststoffe wie Polyacrylate, Citrat und Phosphonate sein. Insbesondere die erfindungsgemäßen Waschmittel können zusätzliche Additive wie Farbschutzmittel, Soil-Release-Polymere, Farbtransferinhibitoren, Entschäumer, Enzyme oder Bleichmittel enthalten.

Die erfindungsgemäßen Wasch- und Reinigungsmittel enthalten die eine oder die mehreren Verbindungen der Komponente a) vorzugsweise in Mengen von 0,01 bis 10,0 Gew.-%, besonders bevorzugt in Mengen von 0,1 bis 5,0 Gew.-%, insbesondere bevorzugt in Mengen von 0,2 bis 3,0 Gew.-% und außerordentlich bevorzugt in Mengen von 0,5 bis 2,0 Gew.-% und die eine oder die mehreren Substanzen der Komponente b) vorzugsweise in Mengen von 0,01 bis 10,0 Gew.-%, besonders bevorzugt in Mengen von 0,1 bis 5,0 Gew.-%, insbesondere bevorzugt in Mengen von 0,2 bis 3,0 Gew.-% und außerordentlich bevorzugt in Mengen von 0,5 bis 2,0 Gew.-%, jeweils bezogen auf das Gesamtgewicht der erfindungsgemäßen Zusammensetzung.

Wie bereits erwähnt enthalten die erfindungsgemäßen Wasch- und Reinigungsmittel in einer bevorzugten Ausführungsform der Erfindung keine Verbindungen ausgewählt aus Sorbitol und Sorbitolestern. Sofern die erfindungsgemäßen Wasch- und Reinigungsmittel jedoch eine oder mehrere Verbindungen ausgewählt aus Sorbitol und Sorbitolestern (wobei die der Säurekomponente dieser Ester zu Grunde liegende Carbonsäure vorzugsweise Caprylsäure ist) enthalten, sind diese Verbindungen gemeinsam vorzugsweise in einer Menge kleiner oder gleich 0,1 Gew.-%, besonders bevorzugt in einer Menge kleiner oder gleich 0,06 Gew.-%, insbesondere bevorzugt in einer Menge kleiner oder gleich 0,02 Gew.-% und außerordentlich bevorzugt in einer Menge kleiner oder gleich 0,01 Gew.-% in den erfindungsgemäßen Wasch- und Reinigungsmitteln enthalten, wobei die Angaben in Gew.-% jeweils auf das Gesamtgewicht der fertigen

erfindungsgemäßen Zusammensetzung bezogen sind.

Wie bereits erwähnt enthalten die erfindungsgemäßen Wasch- und Reinigungsmittel in einer weiteren bevorzugten Ausführungsform der Erfindung keine Verbindungen ausgewählt aus Sorbitan und Sorbitanestern. Sofern die erfindungsgemäßen Wasch- und Reinigungsmittel jedoch eine oder mehrere Verbindungen ausgewählt aus Sorbitan und Sorbitanestern (wobei die der Säurekomponente dieser Ester zu Grunde liegende Carbonsäure vorzugsweise Caprylsäure ist) enthalten, sind diese Verbindungen gemeinsam vorzugsweise in einer Menge kleiner oder gleich 0,4 Gew.-%, besonders bevorzugt in einer Menge kleiner oder gleich 0,2 Gew.-%, insbesondere bevorzugt in einer Menge kleiner oder gleich 0,1 Gew.-% und außerordentlich bevorzugt in einer Menge kleiner oder gleich 0,02 Gew.-% in den erfindungsgemäßen Wasch- und Reinigungsmittel enthalten, wobei die Angaben in Gew.-% jeweils auf das Gesamtgewicht der fertigen erfindungsgemäßen Zusammensetzung bezogen sind.

In einer weiteren bevorzugten Ausführungsform der Erfindung haben die erfindungsgemäßen Wasch- und Reinigungsmittel Viskositäten vorzugsweise im Bereich von 50 bis 200 000 mPa · s, besonders bevorzugt im Bereich von 500 bis 100 000 mPa · s, insbesondere bevorzugt im Bereich von 2 000 bis 50 000 mPa · s und außerordentlich bevorzugt im Bereich von 5 000 bis 30 000 mPa · s (20 °C, Brookfield RVT, RV-Spindel-Satz bei 20 Umdrehungen pro Minute).

In einer weiteren bevorzugten Ausführungsform der Erfindung liegen die erfindungsgemäßen Wasch- und Reinigungsmittel in Form von Fluids, Gelen, Schäumen, Sprays, Lotions oder Cremes vor.

Die erfindungsgemäßen Wasch- und Reinigungsmittel sind vorzugsweise auf wässriger oder wässrig-alkoholischer Basis aufgebaut oder liegen als Lösungen, Emulsionen oder Dispersionen vor. Besonders bevorzugt liegen sie als Emulsionen vor und insbesondere bevorzugt liegen sie als Öl-in-Wasser Emulsionen vor.

Die erfindungsgemäßen Wasch- und Reinigungsmittel besitzen pH-Werte von vorzugsweise 2 bis 11, besonders bevorzugt von 4,5 bis 8,5 und insbesondere bevorzugt von 5,5 bis 8.

Zusammensetzungen nach Anspruch 1 bzw. erfindungsgemäße Vormischungen sind in vorteilhafter Weise zum Konservieren von kosmetischen, dermatologischen oder pharmazeutischen Zusammensetzungen, Pflanzenschutzformulierungen oder Wasch- oder Reinigungsmitteln geeignet.

Weiterer Gegenstand der vorliegenden Erfindung ist daher die Verwendung von Zusammensetzungen nach Anspruch 1 bzw. von erfindungsgemäßen Vormischungen zum Konservieren von kosmetischen, dermatologischen oder pharmazeutischen Zusammensetzungen, Pflanzenschutzformulierungen oder Wasch- oder Reinigungsmitteln. Die kosmetischen, dermatologischen oder pharmazeutischen Zusammensetzungen, Pflanzenschutzformulierungen oder Wasch- oder Reinigungsmittel werden hierbei vorzugsweise gegen Bakterien, Hefen und Pilze konserviert. Besonders bevorzugt werden die kosmetischen, dermatologischen oder pharmazeutischen Zusammensetzungen, Pflanzenschutzformulierungen oder Wasch- oder Reinigungsmittel gegen Hefen und Pilze konserviert und insbesondere bevorzugt gegen Pilze.

Die nachfolgenden Beispiele und Anwendungen sollen die Erfindung näher erläutern, ohne sie jedoch darauf zu beschränken. Bei allen Prozentangaben handelt es sich um Gewichts-% (Gew.-%), sofern nicht explizit anders angegeben.

Versuchsbeispiele:

A) Herstellung von Isosorbidcaprylat

In einer Rührapparatur mit Destillationsaufsatz werden 190,0 g (1,3 mol) Isosorbid ("Sorbon" von Ecogreen Oleochemicals) und 187,5 g (1,3 mol) Octansäure (Caprylsäure) bei 80 °C zusammen mit 0,38 g Natronlauge (18 Gew.-%ig, wässrig) als Katalysator vorgelegt. Unter Rühren und Stickstoffüberleitung (10 - 12 Liter pro Stunde) wird das Reaktionsgemisch zunächst auf 180 °C aufgeheizt, wobei das Reaktionswasser abzudestillieren beginnt. Der Ansatz wird dann in 1 Stunde auf 190 °C und in weiteren 2 Stunden auf 210 °C aufgeheizt. Nach Erreichen von 210 °C wird solange verestert bis eine Säurezahl von < 1 mg KOH/g erreicht ist. Man erhält 345,7 g bernsteinfarbenes Isosorbidcaprylat (97 % der Theorie). Der pH-Wert (5 Gew.-% in Ethanol/Wasser 1:1) beträgt 5,9. Der pH-Wert wurde gemessen gemäß DIN EN 1262.

Weitere analytische Kenndaten des Isosorbidcaprylats:

| | |
|---|---|
| Säurezahl: | 0,9 mg KOH/g, gemessen gemäß DIN EN ISO 2114 |
| Hydroxyl-Zahl: | 206 mg KOH/g, gemessen in Anlehnung an DIN 53240-2 nach Methode OHZ-A |
| Verseifungszahl: | 204 mg KOH/g, gemessen gemäß DIN EN ISO 3681 |

Das Isosorbidcaprylat besitzt folgende Zusammensetzung:

| Substanz | Gew.-% |
|---|---|
| Caprylsäure | 0,4 |
| Isosorbid | 18,1 |
| Isosorbidmonocaprylat | 50,9 |
| Isosorbiddicaprylat | 30,6 |

Diese Zusammensetzung wird im Folgenden als "Isosorbidcaprylat 1" bezeichnet.

B) Bestimmung der antimikrobiellen Wirksamkeit erfindungsgemäßer Zusammensetzungen

Im Folgenden wird die antimikrobielle Wirksamkeit einer erfindungsgemäßen Zusammensetzung bestehend aus 50 Gew.-% Isosorbidcaprylat 1 und 50 Gew.-% Ethylhexylglycerin gegen Bakterien, Pilze und Hefen untersucht (die Zusammensetzung wird im Folgenden "Zusammensetzung A" genannt). Für die Ausprüfung mit Bakterien wurde Zusammensetzung A mit Butylpolyglykol verdünnt und anschließend zu flüssigem, auf pH 7 (+/- 0,2) gepufferten Caso-Agar (Casein-Pepton-Agar) bei 50 °C in unterschiedlichen Konzentrationen gegeben (im Folgenden Zusammensetzungen B1, B2, etc. genannt). Für die Ausprüfung mit Pilzen und mit Hefen wurde Zusammensetzung A mit Butylpolyglykol verdünnt und anschließend zu flüssigem, auf pH 5,6 (+/- 0,2) gepufferten Sabouraud-4 %-Dextrose Agar in unterschiedlichen Konzentrationen gegeben (im Folgenden Zusammensetzungen PH1, PH2, etc. genannt). Jede der Zusammensetzungen B1, B2, etc. bzw. PH1, PH2 etc. wurde in Petrischalen ausgegossen und jeweils mit der gleichen Menge an Bakterien, Pilzen und Hefen beimpft. Die minimale Hemmkonzentration (MHK) ist die Konzentration, bei der eine Hemmung des Wachstums der Bakterien, Pilze und Hefen in den Zusammensetzungen B1, B2, etc. bzw. PH1, PH2, etc. auftritt.

Analog wurden die minimalen Hemmkonzentrationen für die reinen Substanzen Isosorbidcaprylat 1 und Ethylhexylglycerin bestimmt.

Die ermittelten und im Folgenden in Tabelle 1 angegebenen Werte für die minimalen Hemmkonzentrationen "MHK Mischung" sind auf die Konzentrationen der Zusammensetzung A bezogen.

Die ermittelten und im Folgenden in Tabelle 1 angegebenen Werte für die minimalen Hemmkonzentrationen "$Q_A$" und "$Q_B$" sind bereits um den Verdünnungseffekt des Butylpolyglykols bereinigt.

Aus den ermittelten minimalen Hemmkonzentrationen kann dann ausgerechnet werden, ob ein synergistischer Effekt vorliegt oder nicht. Ob ein synergistischer Effekt vorliegt wird nach F.C. Kull et al., Applied Microbiology 1961, 9, 538 mit folgender Formel berechnet:

$$SE = Q_a/Q_A + Q_b/Q_B$$

wobei

$Q_a$ die minimale Hemmkonzentration von Isosorbidcaprylat 1 in der eingesetzten Mischung ist,
$Q_a$ die minimale Hemmkonzentration von Isosorbidcaprylat 1 ist,
$Q_b$ die minimale Hemmkonzentration von Ethylhexylglycerin in der eingesetzten Mischung ist und
$Q_B$ die minimale Hemmkonzentration von Ethylhexylglycerin ist.

Die Werte für $Q_a$ und $Q_b$ werden aus den Werten für die Mischungen ("MHK Mischung") errechnet, indem die ermittelten minimalen Hemmkonzentrationen aufgrund der Anteile der Inhaltsstoffe in der untersuchten erfindungsgemäßen Zusammensetzung A aus 50 Gew.-% Isosorbidcaprylat 1 und 50 Gew.-% Ethylhexylglycerin für $Q_a$ mit dem Faktor 0,5 und für $Q_b$ mit dem Faktor 0,5 multipliziert werden.

Wird ein SE-Wert > 1 erhalten, so liegt eine antagonistische Wirkung vor. Ist SE = 1, so verhalten sich die Verbindungen gegenüber einander neutral und ist SE < 1 liegt ein synergistischer Effekt vor.

In der folgenden Tabelle 1 sind die Ergebnisse der Untersuchung der Zusammensetzung A wiedergegeben.

Tabelle 1 Ergebnisse zur Untersuchung der antimikrobiellen Wirksamkeit der Zusammensetzung A

| Untersuchte Bakterien (B), Pilze (P) oder Hefen (H) | MHK Mischung, gem. [ppm] | $Q_a$, ber. [ppm] | $Q_A$, gem. [ppm] | $Q_b$, ber. [ppm] | $Q_B$, gem. [ppm] | SE |
|---|---|---|---|---|---|---|
| *Staphylococcus aureus* (B) | 1000 | 500 | 2000 | 500 | 750 | 0,92 |
| *Pseudomonas aeruginosa* (B) | 5000 | 2500 | 10000 | 2500 | 5000 | 0,75 |
| *Escherichia coli* (B) | 1500 | 750 | 2000 | 750 | 1000 | 1,13 |
| *Enterobacter aerogenes* (B) | 5000 | 2500 | 10000 | 2500 | 2000 | 1,5 |
| *Klebsiella pneumoniae* (B) | 2000 | 1000 | 10000 | 1000 | 2000 | 0,6 |
| *Proteus vulgaris* (B) | 1500 | 750 | 10000 | 750 | 1500 | 0,58 |
| *Pseudomonas oleovorans* (B) | 10000 | 5000 | 10000 | 5000 | 5000 | 1,5 |
| *Citrobacter freundii* (B) | 1500 | 750 | 10000 | 750 | 1500 | 0,58 |
| *Candida albicans* (H) | 750 | 375 | 750 | 375 | 1000 | 0,88 |
| *Aspergillus brasiliensis* (P) | 750 | 375 | 1000 | 375 | 1000 | 0,75 |
| *Penicillium minioluteum* (P) | 500 | 250 | 750 | 250 | 750 | 0,66 |
| *Aspergillus terreus* (P) | 500 | 250 | 750 | 250 | 1000 | 0,58 |
| *Fusarium solani* (P) | 250 | 125 | 750 | 125 | 500 | 0,42 |
| *Penicillium funicolosium* (P) | 250 | 125 | 500 | 125 | 500 | 0,5 |
| gem.: gemessen; ber.: berechnet | | | | | | |

An den in Tabelle 1 aufgeführten Ergebnissen erkennt man, dass eine erfindungsgemäße Zusammensetzung bestehend aus 50 Gew.-% Isosorbidcaprylat 1 und 50 Gew.-% Ethylhexylglycerin insbesondere für die getesteten Hefen und Pilze einen synergistischen Effekt in Bezug auf ihre antimikrobielle Wirksamkeit zeigt.

C) Antimikrobielle Wirksamkeit der Bestandteile von Isosorbidcaprylat 1

Caprylsäure besitzt eine antimikrobielle Wirksamkeit. Da Caprylsäure in der Zusammensetzung "Isosorbidcaprylat 1" aber nur zu 0,4 Gew.-% vorliegt, ist ihre antimikrobielle Wirksamkeit in dieser Zusammensetzung vernachlässigbar klein. Caprylsäure hat zudem bei pH-Werten von 6 oder größer keine antimikrobielle Wirksamkeit.

Analog zur Bestimmung der antimikrobiellen Wirksamkeit gemäß obigem Beispiel B) wurde in weiteren Testreihen die antimikrobielle Wirksamkeit einer Mischung enthaltend 89,6 Gew.-% Isosorbiddicaprylat und 9,4 Gew.-% Isosorbidmonocaprylat (Rest: 1 Gew.-%) (im Folgenden "Isosorbiddicaprylat" genannt) einerseits und reinem Isosorbid andererseits bestimmt. Die Ergebnisse sind in Tabelle 2 dargestellt.

Tabelle 2 Minimale Hemmkonzentrationen (MHK) von Isosorbiddicaprylat und Isosorbid

| Untersuchte Bakterien (B), Pilze (P) oder Hefen (H) | MHK von Isosorbiddicaprylat [ppm] | MHK von Isosorbid [ppm] |
|---|---|---|
| *Staphylococcus aureus (B)* | 10000 | 10000 |
| *Pseudomonas aeruginosa (B)* | 10000 | 10000 |
| *Escherichia coli (B)* | 10000 | 10000 |
| *Enterobacter aerogenes (B)* | 10000 | 10000 |
| *Klebsiella pneumoniae (B)* | 10000 | 10000 |
| *Proteus vulgaris (B)* | 10000 | 10000 |
| *Pseudomonas oleovorans (B)* | 10000 | 10000 |
| *Citrobacter freundii (B)* | 10000 | 10000 |
| *Candida albicans (H)* | 10000 | 10000 |

(fortgesetzt)

| Untersuchte Bakterien (B), Pilze (P) oder Hefen (H) | MHK von Isosorbiddicaprylat [ppm] | MHK von Isosorbid [ppm] |
|---|---|---|
| *Aspergillus brasiliensis (P)* | 10000 | 10000 |
| *Penicillium minioluteum (P)* | 10000 | 10000 |
| *Aspergillus terreus (P)* | 10000 | 10000 |
| *Fusarium solani (P)* | 5000 | 10000 |
| *Penicillium funicolosium (P)* | 5000 | 10000 |

Wie aus den Ergebnissen der Tabelle 2 hervorgeht, besitzen weder Isosorbid noch Isosorbiddicaprylat eine antimikrobielle Wirksamkeit.

Auf Grund der fehlenden antimikrobiellen Wirksamkeit der in der Zusammensetzung Isosorbidcaprylat 1 enthaltenen Verbindungen Caprylsäure, Isosorbid und Isosorbiddicaprylat einerseits und der aus den Ergebnissen der Tabelle 1 ersichtlichen antimikrobiellen Wirksamkeit der Zusammensetzung "Isosorbidcaprylat 1" andererseits (siehe minimale Hemmkonzentration $Q_A$ für Isosorbidcaprylat 1 in Tabelle 1),
wird gefolgert, dass die ebenfalls in der Zusammensetzung Isosorbidcaprylat 1 enthaltene Verbindung Isosorbidmonocaprylat eine signifikante antimikrobielle Wirksamkeit besitzt.

Aus diesem Grund wird auch davon ausgegangen, dass die geringfügige Aktivität von der Zusammensetzung Isosorbiddicaprylat gegenüber den Pilzen *Fusarium solani* und *Penicillium funicolosium* auf die darin enthaltene Verbindung Isosorbidmonocaprylat zurückzuführen ist.

D) Anwendungsbeispiele

I) Beispiele zu erfindungsgemäßen Zusammensetzungen

Beispiele a) - d)

Zusammensetzungen bestehend aus

a) 50 Gew.-% Isosorbidcaprylat 1, 50 Gew.-% Ethylhexylglycerin
b) 25 Gew.-% Isosorbidcaprylat 1, 75 Gew.-% Ethylhexylglycerin
c) 75 Gew.-% Isosorbidcaprylat 1, 25 Gew.-% Ethylhexylglycerin
d) 40 Gew.-% Isosorbidcaprylat 1, 20 Gew.-% Ethylhexylglycerin, 20 Gew.-% Benzylalkohol, 20 Gew.-% Benzoesäure

Die Herstellung der Zusammensetzungen der Beispiele a) bis d) erfolgt, indem die einzelnen Komponenten unter Rühren nacheinander am Fingerrührer bei Rührgeschwindigkeiten von 200 - 300 Umdrehungen/Minute unter Vorlage von auf 80 °C erwärmtem flüssigem Isosorbidcaprylat 1 vermengt werden.

II) Beispiele zu erfindungsgemäßen kosmetischen Formulierungen

Folgende kosmetische Formulierungen, Pflanzenschutzformulierungen sowie Wasch- und Reinigungsmittel 1 - 38 werden mit erfindungsgemäßen Zusammensetzungen der Beispiele a) - d) hergestellt:

Formulierungsbeispiele 1 - 4: Haarpflegegele für starken Halt und exzellentes Styling

| Formulierung Nr. | 1 | 2 | 3 | 4 |
|---|---|---|---|---|
| Inhaltsstoff | Menge des jeweiligen Inhaltsstoffs [Gew.-%] | | | |
| Aristoflex® AVC | 1,0 | 1,0 | 1,0 | 1,0 |
| Wasser | ad 100 | ad 100 | ad 100 | ad 100 |
| Carbomer | - | 0,5 | 0,5 | - |
| NaOH | - | q.s. | q.s. | - |
| PEG-40 Hydrogenated Castor Oil | 1,0 | 1,0 | 1,0 | - |

(fortgesetzt)

| Formulierung Nr. | 1 | 2 | 3 | 4 |
|---|---|---|---|---|
| Fragrance | 0,3 | 0,3 | - | 0,3 |
| Ethanol (96 Gew.-% in Wasser) | 10,0 | 10,0 | 5,0 | - |
| Diaformer® Z-712 N (Acrylates/Lauryl acrylates/Stearyl Acrylate/Ethylamine Oxide Methacrylate) | 4,5 | 4,5 | - | 6,0 |
| Luviskol® VA 64 (PVP/VA) | 3,0 | 3,0 | 5,0 | - |
| Propylene Glycol | 1,0 | 1,0 | - | 1,0 |
| Panthenol | 0,5 | 0,5 | - | - |
| Dyestuff solution | q.s. | q.s. | q.s. | - |
| Erfindungsgemäßes Beispiel a) - d) | 0,8 | 0,8 | 0,5 | 0,7 |

Herstellung:

Aristoflex® AVC wird in Wasser gelöst. Im Falle von Carbomerzugabe wird anschließend mit NaOH auf pH = 7 neutralisiert. Die übrigen Komponenten werden gegebenenfalls mit PEG-40 Hydrogenated Castor Oil gemischt und in die verdickte Wasserphase eingerührt.

Formulierungsbeispiel 5: O/W Exfoliating Creme mit hohem Elektrolytgehalt (Na-Glykolat)

| Phase | Inhaltsstoff | Gew.-% |
|---|---|---|
| A | PEG-120 Methyl Glucose Dioleat | 1,5 |
| B | Wasser | ad 100 |
| C | Mineral Oil<br>Caprylyl Trimethicone | 5,0<br>3,0 |
| D | Aristoflex® AVC | 1,2 |
| E | Glykolsäure 30 Gew.-% in Wasser (neutralisiert mit NaOH zu pH = 4)<br>Erfindungsgemäßes Beispiel a) - d) | 6,0<br><br>0,6 |
| F | Laureth-7 | 3,0 |

Herstellung:

A unter Erwärmen in Phase B lösen. Phase C in Phase D dispergieren und in die Wasserphase einrühren. Anschließend Phasen E und F einrühren.

Formulierungsbeispiel 6: W/O Pflege-Hautmilch

| Phase | Inhaltsstoff | Gew.-% |
|---|---|---|
| A | Amodimethicone Glycerocarbamate<br>Cyclopentasiloxane<br>Paraffin Oil<br>Apricot Kernel Oil<br>Grape Seed Oil<br>Microcrystalline Wax<br>Stearic acid<br>Ethylhexyl Cocoate | 2,0<br>5,0<br>3,5<br>1,0<br>0,5<br>0,7<br>0,5<br>7,0 |
| B | Aristoflex® AVC | 0,3 |

(fortgesetzt)

| Phase | Inhaltsstoff | Gew.-% |
|---|---|---|
| C | Wasser<br>Glycerin<br>Erfindungsgemäßes Beispiel a) - d) | ad 100<br>3,5<br>0,5 |

Herstellung:

Die Ölphase A auf 80 °C erhitzen und das Polymer B einrühren. Phase C langsam in kleinen Portionen unter starkem Rühren zugeben und auf Raumtemperatur abkühlen lassen.

Formulierungsbeispiel 7: Makeup-Remover mit exzellenten Hautgefühl

| Phase | Inhaltsstoff | Gew.-% |
|---|---|---|
| A | Isopropyl $C_{12-15}$ Pareth-9 carboxylate | 5,0 |
| B | Sodium Cocoyl Glutamate<br>(25 Gew.-%ige Lösung in Wasser)<br>Cocamidopropyl Betaine<br>(30 Gew.-%ige Lösung in Wasser)<br>Laureth-7<br>Wasser<br>Allantoin<br>Polypropylene Terephthalate<br>1,6 Hexanediol<br>Propylene Glycol<br>PEG-8<br>Panthenol<br>Poloxamer 407<br>Erfindungsgemäßes Beispiel a) - d)<br>Aristoflex® HMB | 2,3<br><br>3,0<br><br>2,0<br>ad 100<br>0,3<br>1,0<br>2,0<br>2,0<br>2,0<br>0,5<br>3,0<br>0,8<br>1,0 |

Herstellung:

Die Komponenten von B nacheinander in A lösen

Formulierungsbeispiel 8: Shampoo/Duschbad mit suspendierten Partikeln

| Phase | Inhaltsstoff | Gew.-% |
|---|---|---|
| A | Wasser | ad 100 |
| B | Aristoflex® TAC | 2,0 |
| C | Sodium Laureth Sulfate (30 Gew.-% in Wasser)<br>Parfüm | 18,5<br>0,5 |
| | Erfindungsgemäßes Beispiel a) - d) | 0,4 |
| D | Sodium Cocoyl Glutamate (25 Gew.-%ige Lösung in Wasser) | 20,0 |
| E | Synthetic Wax | 0,2 |

Herstellung:

Aristoflex® TAC in Wasser lösen, dann nacheinander Phasen C, D und E eintragen und homogenisieren.

Formulierungsbeispiel 9: Klares deodorierendes Gel

| Phase | Inhaltsstoff | Gew.-% |
|---|---|---|
| A | PEG-40 hydrogenated Castor Oil<br>Parfum | 1,0<br>0,1 |
| B | Ethanol (96 Gew.-% in Wasser)<br>Erfindungsgemäßes Beispiel a) - d) | 25,0<br>0,4 |
| C | Propylene Glycol<br>Diisopropyl Adipate<br>Wasser | 20,0<br>1,0<br>ad 100 |
| D | Aristoflex® AVC | 1,3 |
| E | Zitronensäure | q.s. |

Herstellung:

Phase A wird gemischt, sodann werden Phase B und Phase C nacheinander zugegeben und der pH-Wert mit Phase E auf 5,5 angepasst. Abschließend wird Phase D eingerührt, bis ein homogenese klares Gel entsteht.

Formulierungsbeispiel 10: Mattierendes Serum

| Phase | Inhaltsstoff | Gew.-% |
|---|---|---|
| A | Wasser | ad 100 |
| B | Glycerin<br>Aristoflex® HMB<br>Caprylyl Methicone<br>Cyclomethicone und Dimethicone Crosspolymer (Dow Corning 9040 Silicone Elastomer blend)<br>Fragrance<br>Erfindungsgemäßes Beispiel a) - d) | 3,0<br>0,5<br>1,5<br>1,0<br>0,15<br>0,4 |

Herstellung:

Die Komponenten von B werden nacheinander in Phase A eingerührt.

Formulierungsbeispiel 11: Skin Whitening Gel

| Phase | Inhaltsstoff | Gew.-% |
|---|---|---|
| A | Allantoin | 0,5 |
| B | Wasser | ad 100 |
| C | Xanthan Gum | 0,5 |
| D | Ascorbic Acid 2-Glucoside | 2,0 |
| E | NaOH (25 Gew-%ige Lösung in Wasser) | q.s. |
| F | Glycerin<br>Ethanol (96 Gew.-% in Wasser)<br>PEG/PPG-18/18 Dimethicone (Dow Corning® 190, Dow Corning)<br>PEG-40 hydrogenated Castor Oil | 10,0<br>10,0<br>1,0<br>0,8 |
| G | Aristoflex® AVS | 1,0 |
| H | NaOH (25 Gew-%ige Lösung in Wasser) | q.s. |
| I | Erfindungsgemäßes Beispiel a) - d) | 0,6 |

Herstellung:

Phase A wird in Phase B unter Erwärmen gelöst, Phase C eingerührt, Phase D zugegeben und mit Phase E auf pH = 6,5 eingestellt. Phase F wird gemischt und dann zugegeben, anschließend wird Phase G zugegeben und gerührt, bis ein homogenes Gel erzielt wird. Mit Phase H wird der pH-Wert gegebenenfalls auf 6,5 eingestellt und Phase I eingerührt.

Formulierungsbeispiel 12: Elegante O/W Hautpflege-Bodylotion mit geringer Klebrigkeit

| Phase | Inhaltsstoff | Gew.-% |
|---|---|---|
| A | Caprylic/Capric Triglyceride<br>Myristyl Myristate<br>Cetearyl Alcohol<br>Glyceryl Stearate Citrate<br>Octyldodecanol | 3,5<br>2,5<br>2,0<br>1,0<br>1,0 |
| B | Aristoflex® AVC | 0,6 |
| C | Wasser<br>Glycerin | ad 100<br>7,5 |
| D | Ethanol (96 Gew.-% in Wasser)<br>Dimethicone<br>Tocopheryl Acetate<br>Aloe Barbadensis<br>Erfindungsgemäßes Beispiel a) - d)<br>Fragrance | 3,0<br>3,0<br>1,0<br>1,0<br>0,7<br>q.s. |
| E | NaOH (10 Gew-% in Wasser) | q.s. |

Herstellung:

Phase A wird bei 70 °C aufgeschmolzen, Phase B eingestreut und die auf 70 °C erwärmte Phase C eingerührt. Nach Abkühlen auf 35 °C wird Phase D eingerührt und der pH-Wert abschließend mit Phase E auf 6 eingestellt.

Formulierungsbeispiel 13: Tensidfreie Anti-Ageing O/W Gelcreme mit hautfaltenreduzierender Funktion

| Phase | Inhaltsstoff | Gew.-% |
|---|---|---|
| A | Dicaprylyl Ether<br>Caprylic/Capric Triglyceride<br>Cetearyl Alcohol<br>Erfindungsgemäßes Beispiel a) - d) | 5,0<br>5,0<br>2,0<br>0,6 |
| B | Ubiquinone | 0,1 |
| C | Aristoflex® HMB | 1,1 |
| D | Sodium Hyaluronate (Dekluron)<br>Glycerin | 0,3<br>8,0 |
| E | Wasser<br>Mica und Titanium Dioxide und Tin Oxide (Prestige® Soft Orange, Eckart) | Ad 100<br>0,5 |
| F | Tocopheryl Acetate | 0,3 |
| G | NaOH (10 Gew.-% in Wasser) | q.s |

Herstellung:

Phase A wird bei 80 °C geschmolzen, Phase B und Phase C nacheinander eingerührt. Phase D wird in Phase

E vorgelöst und zugegeben. Phase F wird bei 35 °C eingerührt und mit Phase G der pH-Wert auf 6,0 eingestellt. Es entsteht eine Gelcreme.

Formulierungsbeispiel 14: Tensidfreie Anti-Ageing O/W Gelcreme

| Phase | Inhaltsstoff | Gew.-% |
|---|---|---|
| A | Dicaprylyl Ether<br>Caprylic/Capric Triglyceride<br>Cetearyl Alcohol<br>Erfindungsgemäßes Beispiel a) - d) | 5,0<br>5,0<br>2,0<br>0,8 |
| B | Ubiquinone | 0,1 |
| C | Aristoflex® HMB | 1,1 |
| D | Xanthan Gum<br>Glycerin | 0,2<br>8,0 |
| E | Wasser<br>Mica und Titanium Dioxide und Tin Oxide (Prestige$^{0}$ Soft Orange, Eckart) | ad 100<br>0,5 |
| F | Tocopheryl Acetate | 0,3 |
| G | NaOH (10 Gew.-% in Wasser) | q.s |

Herstellung:

Phase A wird bei 80 °C geschmolzen, Phase B und Phase C nacheinander eingerührt. Phase D wird in Phase E vorgelöst und zugegeben. Phase F wird bei 35 °C eingerührt und mit Phase G der pH-Wert auf 6,0 eingestellt. Es entsteht eine Gelcreme.

Formulierungsbeispiel 15: O/W Selbstbräunungscreme mit Moisturizing-Effekt

| Phase | Inhaltsstoff | Gew.-% |
|---|---|---|
| A | Cetyl Phsophate<br>Glyceryl Stearate<br>Cetearyl Alcohol<br>Isohexadecane<br>Isopropyl Palmitate<br>Caprylyl Methicone | 1,0<br>0,5<br>0,5<br>8,0<br>7,0<br>1,0 |
| B | Aristoflex® AVS | 1,0 |
| C | Wasser<br>Sodium Cocoyl Glutamate<br>Glycerin<br>NaOH (10 Gew.-% in Wasser) | ad 100<br>0,5<br>5,0<br>0,5 |
| D | Tocopheryl Acetate<br>Fragrance<br>Erfindungsgemäßes Beispiel a) - d) | 1,0<br>0,2<br>0,5 |
| E | Dihydroxyacetone<br>Wasser | 5,0<br>8,0 |

Herstellung:

Phase A wird bei 80 °C geschmolzen, Phase B und Phase C nacheinander eingerührt. Phase D wird bei 30 °C zugeben und Phase E abschließend eingerührt. Es resultiert eine Creme mit einen pH-Wert von 4,2.

Formulierungsbeispiel 16 - 21: W/O Sonnenschutzformulierungen mit hohem Schutzfaktor

| Formulierung Nr. | 16 | 17 | 18 | 19 | 20 | 21 |
|---|---|---|---|---|---|---|
| Inhaltsstoff | Menge des jeweiligen Inhaltsstoffs [Gew.-%] | | | | | |
| $C_{12-15}$ Alkyl benzoate | 8,0 | 8,0 | 8,0 | 8,0 | 8,0 | 8,0 |
| Caprylic Capric Triglyceride | 5,0 | 5,0 | 5,0 | 5,0 | 5,0 | 5,0 |
| Octocrylene | 9,0 | - | 5,0 | 4,0 | - | - |
| Ethylhexyl Methoxycinnamate | 7,0 | 7,0 | 7,0 | - | 6,0 | 6,0 |
| Butyl Methoxydibenzoylmethane | 2,5 | - | 2,5 | - | - | - |
| Disodium Phenyl Dibenzimidazole Tetrasulfonate | - | - | - | - | - | 3,0 |
| Ethylhexyl Bis-Isopentylbezoxazolylphenylmelamine | - | - | - | - | 2,0 | - |
| Diethylamino Hydroxybenzoyl Hexyl Benzoate | - | - | 2,0 | 1,0 | - | - |
| Bis Ethylhexyloxyphenol Methoxyphenyl Triazine | - | 3,0 | - | 2,0 | 4,0 | 3,0 |
| Methylene Bis-Benzotriazolyl Tetramethylbutylphenol | - | 3,0 | - | - | - | 2,0 |
| Ethylhexyl Triazone | - | - | - | 3,0 | - | - |
| Diethylhexyl Butamido Triazone | - | - | - | - | 2,0 | - |
| Polysilicone-15 | - | - | 2,0 | - | - | - |
| Phenylbenzimidazole Sulfonic Acid | - | - | - | 3,0 | - | - |
| Titanium Dioxide | - | 5,0 | 3,0 | 4,0 | 5,0 | 5,0 |
| Cetearyl Alcohol | 1,0 | 1,0 | 1,0 | 1,0 | 1,0 | 1,0 |
| Sunflower Seed Oil Sorbitol Esters | 2,0 | 2,0 | 2,0 | 2,0 | 2,0 | 2,0 |
| Erfindungsgemäßes Beispiel a) - d) | 0,8 | 0,8 | 0,8 | 0,8 | 0,8 | 0,8 |
| Kaliumcetylphosphat | 3,0 | 3,0 | 3,0 | 3,0 | 3,0 | 3,0 |
| Aristoflex® AVC | 1,0 | 0,6 | 0,5 | 0,9 | 1,0 | 1,0 |
| Wasser | ad 100 | ad 100 | ad 100 | ad 100 | ad 100 | ad 100 |
| Nylon | - | 0,5 | - | - | - | - |
| Bis-Ethylhexyl Hydroxydimethoxy Benzylmalonate | - | - | 1,0 | - | - | - |
| Talc | - | - | - | - | 0,5 | - |

Herstellung:

Zur Herstellung wurden die öllöslichen Komponenten auf 80 °C erhitzt, Kaliumcetylphosphat sowie Aristoflex® AVC eingestreut und die vereinigten wasserlöslichen Phasen langsam unter starkem Rühren in die Ölphase eingetragen. Die gebildeten Emulsionen wurden unter Rühren auf Raumtemperatur abkühlen lassen.

Die in den Formulierungsbeispielen 16-21 verwendeten Sonnenschutzfilter, ihre Markennamen sowie ihr UV-Schutzbereich sind in der folgenden Tabelle aufgeführt.

| Sonnenschutzfilter | Markenname | Schutzbereich (UV-A/UV-B) |
|---|---|---|
| Octocrylene | Neo Heliopan® 303 | B |
| Ethylhexyl Methoxycinnamate | Neo Heliopan® AV | B |
| Butyl Methoxydibenzoylmethane | Neo Heliopan® 357, Parsol® 1789 | A |

(fortgesetzt)

| Sonnenschutzfilter | Markenname | Schutzbereich (UV-A/UV-B) |
|---|---|---|
| Disodium Phenyl Dibenzimidazole Tetrasulfonate | Neo Heliopan® AP | A |
| Ethylhexyl Bis-Isopentylbezoxazolylphenyl-melamine | Uvasorb® K2A | A |
| Diethylamino Hydroxybenzoyl Hexyl Benzoate | Uvinul® A Plus | A |
| Bis Ethylhexyloxyphenol Methoxyphenyl Triazine | Tinosorb® S | A/B |
| Methylene Bis-Benzotriazolyl Tetramethylbutylphenol | Tinosorb® M | A/B |
| Ethylhexyl Triazone | Uvinul® T 150 | B |
| Diethylhexyl Butamido Triazone | Uvasorb® HEB | B |
| Polysilicone-15 | Parsol® SLX | B |
| Phenylbenzimidazole Sulfonic Acid | | B |

Formulierungsbeispiel 22: O/W Sonnenschutzcreme

| Phase | Inhaltsstoff | Gew.-% |
|---|---|---|
| A | Ethylhexyl Methoxycinnamate<br>Ethylhexyltriazone<br>Benzophenone-3<br>BHT | 6,0<br>2,0<br>2,0<br>0,05 |
| B | Aristoflex® AVS<br>Trilaureth-4 Phosphate<br>Polyglyceryl-2 Sesquiisostearate<br>Caprylyl Methicone<br>Erfindungsgemäßes Beispiel a) - d)<br>PVP/Hexadecene Copolymer<br>Tocopheryl Acetate<br>Fragrance | 1,5<br>2,0<br>1,0<br>1,0<br>0,7<br>1,0<br>0,5<br>0,2 |
| C | Wasser<br>Disodium EDTA | ad 100<br>0,1 |
| D | Methylene Bis-Benzotriazolyl Tetramethylbutylphenol | 4,0 |
| E | Triethanolamin | q.s. |

Herstellung:

Phase A homogensieren und bei 60 °C lösen und in Phase B einrühren, dann Phase C unter Rühren zugeben und bei 300 Umdrehungen pro Minute rühren. Anschließend wird Phase D eingerührt und mit E der pH-Wert auf 6,8 - 7,2 eingestellt.

Formulierungsbeispiel 23: Sprühbare O/W Lotion

| Phase | Inhaltsstoff | Gew.-% |
|---|---|---|
| A | Trilaureth-4 Phosphate<br>Mineral Oil<br>Isopropyl Palmitate<br>Cetearyl Alcohol | 1,0<br>8,0<br>3,0<br>0,5 |

(fortgesetzt)

| Phase | Inhaltsstoff | Gew.-% |
|---|---|---|
| | Caprylic/Capric Triglyceride<br>Glyceryl Stearate<br>Caprylyl Methicone | 2,0<br>0,5<br>1,0 |
| B | Aristoflex® AVC | 0,2 |
| C | Wasser<br>Glycerin | ad 100<br>5,0 |
| D | Fragrance<br>Ethanol (96 Gew.-% in Wasser) | 0,3<br>5,0 |
| E | Erfindungsgemäßes Beispiel a) - d) | 0,6 |

Herstellung:

Phase A auf 60 °C erhitzen, Phase B einrühren, dann Phase C unter Rühren zugeben und bei 300 Umdrehungen pro Minute rühren und abkühlen lassen. Phase D bei 35 °C einrühren, Phase E zugeben und schließlich homogenisieren.

Formulierungsbeispiel 24: O/W Foundation

| Phase | Inhaltsstoff | Gew.-% |
|---|---|---|
| A | Hydrogenated Polydecene<br>Caprylic/Capric Triglyceride<br>Caprylyl Trimethicone<br>Caprylyl Methicone<br>Steareth-2<br>Steareth-20<br>Aristoflex® HMB | 9,0<br>5,0<br>4,0<br>3,0<br>1,6<br>2,4<br>0,4 |
| B | Kaolin<br>Talc<br>Iron Oxide | 1,5<br>3,0<br>7,9 |
| C | Glycerin<br>Wasser | 5,0<br>ad 100 |
| D | Erfindungsgemäßes Beispiel a) - d)<br>Fragrance | 0,6<br>q.s. |

Herstellung:

Phase A auf 70 °C erhitzen, Phase C auf 70 °C erhitzen. Phase B in Phase A einrühren, dann Phase C zugeben und gut homogenisieren. Nach Abkühlen unter 40 °C Phase D zugeben und eine Minute homogenisieren.

Formulierungsbeispiel 25: Antischuppen Shampoo

| Phase | Inhaltsstoff | Gew.-% |
|---|---|---|
| A | Erfindungsgemäßes Beispiel a) - d) | 1,0 |
| B | Wasser | 10,0 |
| C | Sodium Laureth Sulfate | 30,0 |
| D | Climbazole | 0,5 |
| E | 1,2-Propylenglykol | 2,0 |
| | Sodium Cocoyl Glutamate | 4,0 |

(fortgesetzt)

| Phase | Inhaltsstoff | Gew.-% |
|---|---|---|
| F | Fragrance<br>Wasser<br>Merquat® 550<br>*Polyquaternium 7*<br>Panthenol<br>Sodium Salicylate<br>Genagen® KB (Clariant)<br>*Coco Betaine*<br>Dyestuff solution | 0,3<br>ad 100<br>0,5<br><br>0,5<br>1,0<br>8,0<br><br>q.s. |
| G | Sodium Chloride | 1,0 |

Herstellung:

I Mische A mit B.
II Gebe C zu I und Rühre bis eine klare Lösung erhalten wird.
III Löse D in E und gebe die Lösung zu II.
IV Rühre die Komponenten von F nacheinander in III.
V Stelle den pH-Wert auf 6,0 - 6,5 ein.
VI Stelle die Viskosität mit G ein.

Formulierungsbeispiel 26: Anti-Akne Gesichtsreiniger

| Phase | Inhaltsstoff | Gew.-% |
|---|---|---|
| A | Genagen® CAB (Clariant)<br>*Cocamidopropyl Betaine* | 10,0 |
| B | Fragrance<br>Hostapon® CLG (Clariant)<br>*Cocoyl Lauroyl Glutamate*<br>Hostapon® CT Paste (Clariant)<br>*Sodium Methyl Cocoyl Taurate*<br>Glycerin<br>Aristoflex® PEA (Clariant)<br>*Polypropylene Terephthalate*<br>Cetiol® HE (Cognis)<br>Erfindungsgemäßes Beispiel a) - d)<br>Aloe Vera-Gel-Konc.<br>*Water (and) Aloe Barbadensis Gel*<br>Extrapon Kamille<br>*Water (and) Ethoxydiglycol (and) Propylene Glycol (and) Matricaria Extract (and) Butylene Glycol (and) Glycose (and) Bisabolol*<br>Wasser<br>D-Panthenol | 0,2<br>2,0<br><br>2,0<br><br>1,0<br>1,0<br><br>1,0<br>1,0<br>1,0<br><br>1,0<br><br>ad 100<br>0,5 |
| C | Zitronensäure | q.s. |

Herstellung:

I Lege A vor und gebe die Komponenten von B nacheinander unter Rühren zu
II Stelle den pH-Wert mit C auf 5,5 - 6,0 ein

Formulierungsbeispiel 27: Kopfhautgel

| Phase | Inhaltsstoff | Gew.-% |
|---|---|---|
| A | Promyristyl® PM-3<br>*PPG-3 Myristyl Ether*<br>Lamesoft® PO 65<br>*Coco-Glucoside (and) Glyceryl Oleate*<br>Cetiol® SB 45<br>*Butyrospermum Parkii (Shea Butter)* | 6,0<br><br>3,0<br><br>2,0 |
| B | Wasser<br>Glycerin<br>Sodium Salicylate<br>Allantoin (Clariant)<br>*Allantoin*<br>Merquat 2001<br>*Polyquaternium-47* | ad 100<br>4,0<br>2,0<br>0,4<br><br>0,5 |
| C | Urea | 10,0 |
| D | Aristoflex® AVC (Clariant)<br>*Ammonium Acyloyldimethyltaurate/VP Copolymer* | 1,8 |
| E | Erfindungsgemäßes Beispiel a) - d) | 0,6 |
| F | Milchsäure | q.s. |

Herstellung

I Mische die Komponenten von A und löse sie bei 50 °C.
II Mische die Komponenten von B unter Rühren und leichtem Erwärmen.
III Löse C bei etwa 25 °C in II.
IV Gebe D zu I.
V Rühre III in IV ein.
VI Gebe E zu.
VII Stelle den pH-Wert mit F auf 5,0 ein.

Formulierungsbeispiel 28: Feuchttuchlösung

| Phase | Inhaltsstoff | Gew.-% |
|---|---|---|
| A | Propylene Glycol<br>Erfindungsgemäßes Beispiel a) - d) | 3,0<br>0,8 |
| B | Wasser<br>Genagen® KB<br>*Coco-Betaine*<br>Genamin® PQ43<br>*Polyquaternium-43*<br>Aristoflex® AVC<br>*Ammonium Acryloyldimethyltaurate/VP Copolymer* | ad 100<br>3,0<br><br>0,7<br><br>0,1 |
| C | Citric acid | q.s. |

I Mische die Komponenten von A.
II Mische die Komponenten von B.
III Gebe II zu I hinzu.
IV Stelle mit C den pH-Wert auf pH 6,0 ein.

Formulierungsbeispiele 29 und 30: Pflanzenschutzformulierungen

| Formulierung Nr. | 29 | 30 |
|---|---|---|
| Inhaltsstoff | Menge des jeweiligen Inhaltsstoffes [Gew.-%] | |
| Atrazin | 43,6 | 43,6 |
| Dispersogen® PSL 100 | - | 1,7 |
| Genapol® LSS | - | 1,6 |
| Dispersogen® LFS | 2,1 | - |
| Propylenglykol | 4,3 | 4,3 |
| Defoamer SE 57 | 0,6 | 0,6 |
| Kelzan® S (2 Gew.-% in Wasser) | 7,3 | 7,3 |
| Erfindungsgemäßes Beispiel a) - d) | 0,5 | 0,5 |
| Wasser | ad 100 | ad 100 |

Herstellung:

Der Wirkstoff wird mit den anderen Inhaltsstoffen (außer der Kelzan® S-Lösung) vordispergiert und anschließend einer Feinmahlung unterzogen, bis die mittlere Teilchengröße < 2 Mikrometer beträgt. Anschließend wird die Kelzan® S-Lösung eingerührt.

Formulierungsbeispiele 31-33 : Handgeschirrspülmittel

| Formulierung Nr. | 31 | 32 | 33 |
|---|---|---|---|
| Inhaltsstoff | Menge des jeweiligen Inhaltsstoffes [Gew. %] | | |
| Hostapur® SAS 60 (Alkansulfonat, 60 Gew.-% in Wasser) | 40,0 | 10,0 | 20,0 |
| Genapol® LRO paste (Ethersulfat mit 2 EO, 70 Gew.-% in Wasser) | 11,0 | 8,5 | 8,5 |
| Genaminox® LA (Dimethyllauraminoxid, 30 Gew.-% in Wasser) | - | - | 3,0 |
| Genagen® CAB (Cocoamidopropylbetain, 30 Gew.-% in Wasser) | 3,0 | 6,0 | - |
| Erfindungsgemäßes Beispiel a) - d) | 0,4 | 0,2 | 0,3 |
| Wasser | ad 100 | ad 100 | ad 100 |

Formulierungsbeispiele 34-37: Oberflächenreiniger (Allzweckreiniger)

| Formulierung Nr. | 34 | 35 | 36 | 37 |
|---|---|---|---|---|
| Inhaltsstoff | Menge des jeweiligen Inhaltsstoffs [Gew.-%] | | | |
| Hostapur® SAS 60 (Alkansulfonat, 60 Gew.-% in Wasser) | 5,0 | - | - | - |
| Genapol® UD 080 (Undecanol + 8 EO) | 2,0 | - | - | - |
| Genaminox® LA (Dimethyllauraminoxid, 30 Gew.-% in Wasser) | - | 2,0 | 6,0 | - |
| Kaliumcocoat (Seife) | - | - | 2,0 | 2,0 |
| Mono-/Triethanolamin 1:1 | - | 1,0 | - | - |
| Natriumcitrat | - | - | 3,0 | 3,0 |

(fortgesetzt)

| Formulierung Nr. | 34 | 35 | 36 | 37 |
|---|---|---|---|---|
| Inhaltsstoff | Menge des jeweiligen Inhaltsstoffs [Gew.-%] | | | |
| Erfindungsgemäßes Beispiel a) - d) | 0,2 | 0,1 | 0,2 | 0,2 |
| Wasser | ad 100 | ad 100 | ad 100 | ad 100 |

Herstellung:

Die Hälfte der Wassermenge wird vorgelegt und die Komponenten werden in der Reihenfolge wie in der Tabelle aufgeführt eingerührt. Die restliche Menge Wasser wird dann nachgegeben. Es resultieren klare, wässrige Reiniger

Formulierungsbeispiel 38: Feinwaschmittel

| Phase | Inhaltsstoff | Gew.-% |
|---|---|---|
| A | Fettsäure<br>Kaliumhydroxid (85 Gew.-% in Wasser) | 3,0<br>0,6 |
| B | Destilliertes Wasser | ad 100 |
| C | Hostapur® SAS 60 (Alkansulfonat, 60 Gew.-% in Wasser)<br>Genapol® LRO liq (Ethersulfat mit 2 EO; 30 Gew.-% in Wasser)<br>Genapol® UD 080 (Undecanol mit 8 EO) | 23,3<br>25,0<br>6,0 |
| D | Texcare® SRN 170 (Soil Release Polymer)<br>Zitronensäure Monohydrat | 1,5<br>0,2 |
| E | Erfindungsgemäßes Beispiel a) - d) | 0,5 |

Herstellung:

I Komponenten A vorlegen.
II B auf 40 - 50 °C erwärmen, zugeben und komplett auflösen.
III C nacheinander zugeben unter gründlichem Rühren.
IV D in der angegebenen Reihenfolge zugeben.
V Abschließend Zugabe von E.

Es resultiert eine leicht trübe Lösung mit dem pH-Wert (1 g/l in Wasser, 20 °C) von 7,5.

Die in den Formulierungsbeispielen 1 - 38 getätigte Angabe "erfindungsgemäßes Beispiel a) - d)" bedeutet, dass jedes der Formulierungsbeispiele 1 - 38 mit jeder einzelnen der Zusammensetzungen gemäß den erfindungsgemäßen Beispielen a) - d) hergestellt werden kann.

**Patentansprüche**

**1.** Zusammensetzung enthaltend

a) eine oder mehrere Verbindungen der Formel (I)

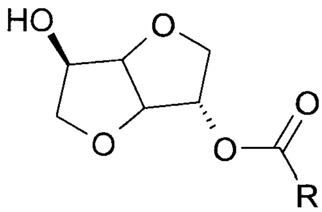

(I)

worin der Rest R in Formel (I) ein linearer gesättigter Alkylrest mit 7 bis 9 Kohlenstoffatomen ist, und
b) ein oder mehrere aliphatische vicinale Diole, ausgewählt aus der Gruppe bestehend aus 1,2-Decandiol, Ethylhexylglycerin, 1,2-Octandiol, 1,2-Hexandiol, Glycerylcaprylat und Glycerylcaproat,
und die Zusammensetzung neben der einen oder den mehreren Verbindungen der Formel (I) eine oder mehrere Verbindungen ausgewählt aus der Gruppe bestehend aus Sorbitol, Sorbitolestern, Sorbitan, Sorbitanestern, Isosorbid, Isosorbiddiestern und Carbonsäuren enthält, wobei die Carbonsäuren selbst als auch die den Säurekomponenten der genannten Ester zu Grunde liegenden Carbonsäuren der Formel RCOOH entsprechen, worin R die bei Formel (I) angegebene Bedeutung besitzt, und die OH-Zahl der Mischung aus der einen oder der mehreren Verbindungen der Formel (I) und der einen oder der mehreren Verbindungen ausgewählt aus der Gruppe bestehend aus Sorbitol, Sorbitolestern, Sorbitan, Sorbitanestern, Isosorbid, Isosorbiddiestern und Carbonsäuren kleiner oder gleich 245 ist.

2. Zusammensetzung nach Anspruch 1, **dadurch gekennzeichnet, dass** der Rest R in Formel (I) ein linearer gesättigter Alkylrest mit 7 Kohlenstoffatomen ist.

3. Zusammensetzung nach Anspruch 1, **dadurch gekennzeichnet, dass** die eine oder mehreren Substanzen der Komponente b) ausgewählt sind aus der Gruppe bestehend aus 1,2-Decandiol, Ethylhexylglycerin, 1,2-Octandiol und 1,2-Hexandiol.

4. Zusammensetzung nach Anspruch 3, **dadurch gekennzeichnet, dass** die Substanz der Komponente b) Ethylhexylglycerin ist.

5. Zusammensetzung nach einem oder mehreren der Ansprüche 1 bis 4, **dadurch gekennzeichnet, dass** sie

I) 0,05 bis 0,7 Gewichtsteile Isosorbid und
II) 0,1 bis 1,0 Gewichtsteile Isosorbiddiester der Formel

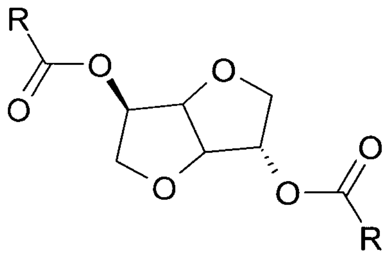

worin R die bei Formel (I) angegebene Bedeutung besitzt,
enthält, jeweils bezogen auf 1,0 Gewichtsteile an der einen oder den mehreren Verbindungen der Formel (I).

6. Zusammensetzung nach einem oder mehreren der Ansprüche 1 bis 5, **dadurch gekennzeichnet, dass** sie zusätzlich einen oder mehrere Sorbitanester aus Sorbitan und Carbonsäuren $R^aCOOH$ enthält, wobei $R^a$ eine lineare oder verzweigte, gesättigte Alkylgruppe mit 5 bis 11 Kohlenstoffatomen oder eine lineare oder verzweigte, ein- oder mehrfach ungesättigte Alkenylgruppe mit 5 bis 11 Kohlenstoffatomen ist, und das Gewichtsverhältnis der einen oder der mehreren Verbindungen der Formel (I) zu dem einen oder den mehreren soeben genannten Sorbitanestern von 70 : 30 bis 100 : 0 ist.

7. Zusammensetzung nach Anspruch 6, **dadurch gekennzeichnet, dass** der eine oder die mehreren Sorbitanester aus Sorbitan und Carbonsäuren $R^aCOOH$ ausgewählt sind aus Sorbitanestern aus Sorbitan und Caprylsäure.

8. Zusammensetzung nach einem oder mehreren der Ansprüche 1 bis 7, **dadurch gekennzeichnet, dass** sie die eine oder die mehreren Verbindungen der Komponente a) in Mengen von 10,0 bis 90,0 Gew.-% und die eine oder die mehreren Substanzen der Komponente b) in Mengen von 10,0 bis 90,0 Gew.-% enthält, jeweils bezogen auf das Gesamtgewicht der Zusammensetzung.

9. Zusammensetzung nach einem oder mehreren der Ansprüche 1 bis 8, **dadurch gekennzeichnet, dass** sie eine kosmetische, dermatologische oder pharmazeutische Zusammensetzung, eine Pflanzenschutzformulierung oder ein Wasch- und Reinigungsmittel ist.

10. Zusammensetzung nach Anspruch 9, **dadurch gekennzeichnet, dass** sie die eine oder die mehreren Verbindungen der Komponente a) in Mengen von 0,01 bis 10,0 Gew.-% und die eine oder die mehreren Substanzen der Komponente b) in Mengen von 0,01 bis 10,0 Gew.-% enthält, jeweils bezogen auf das Gesamtgewicht der Zusammensetzung.

11. Zusammensetzung nach Anspruch 9 oder 10, **dadurch gekennzeichnet, dass** sie auf wässriger oder wässrig-alkoholischer Basis aufgebaut ist oder als Lösung, Emulsion oder Dispersion vorliegt und vorzugsweise als Emulsion vorliegt.

12. Zusammensetzung nach einem oder mehreren der Ansprüche 9 bis 11, **dadurch gekennzeichnet, dass** sie einen pH-Wert von 2 bis 11 besitzt.

13. Verwendung von einer Zusammensetzung nach Anspruch 8 zum Konservieren einer kosmetischen, dermatologischen oder pharmazeutischen Zusammensetzung, einer Pflanzenschutzformulierung oder eines Wasch- oder Reinigungsmittels.

14. Verwendung nach Anspruch 13, **dadurch gekennzeichnet, dass** die kosmetische, dermatologische oder pharmazeutische Zusammensetzung, die Pflanzenschutzformulierung oder das Wasch- oder Reinigungsmittel gegen Bakterien, Hefen und Pilze und vorzugsweise gegenüber Hefen und Pilzen konserviert wird.

## Claims

1. A composition comprising

   a) one or more compounds of the formula (I)

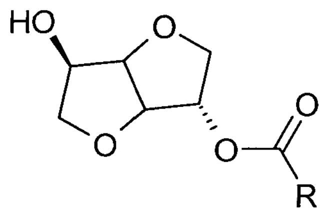

(I)

   in which the radical R in formula (I) is a straight-chain saturated alkyl radical having 7 to 9 carbon atoms, and
   b) one or more aliphatic vicinal diols, selected from the group consisting of 1,2-decanediol, ethylhexylglycerin, 1,2-octanediol, 1,2-hexanediol, glyceryl caprylate and glyceryl caproate,

   and the composition comprises, in addition to the one or more compounds of the formula (I), one or more compounds selected from the group consisting of sorbitol, sorbitol esters, sorbitan, sorbitan esters, isosorbide, isosorbide diesters and carboxylic acids, where the carboxylic acids themselves and the carboxylic acids on which the acid components of the esters mentioned are based correspond to the formula RCOOH in which R has the meaning given for formula (I), and the hydroxyl value of the mixture of the one or more compounds of the formula (I) and the one or more compounds selected from the group consisting of sorbitol, sorbitol esters, sorbitan, sorbitan esters, isosorbide, isosorbide diesters and carboxylic acids is smaller than or equal to 245.

2. The composition as claimed in claim 1, wherein the radical R in formula (I) is a straight-chain saturated alkyl radical having 7 carbon atoms.

**3.** The composition as claimed in claim 1, wherein the one or more substances of component b) are selected from the group consisting of 1,2-decanediol, ethylhexylglycerin, 1,2-octanediol and 1,2-hexanediol.

**4.** The composition as claimed in claim 3, wherein the substance of component b) is ethylhexylglycerin.

**5.** The composition as claimed in one or more of claims 1 to 4, which comprises

I) from 0.05 to 0.7 part by weight of isosorbide and
II) from 0.1 to 1.0 part by weight of isosorbide diester of the formula

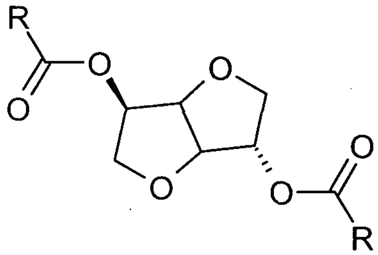

in which R has the meaning given for formula (I), in each case based on 1.0 part by weight of the one or more compounds of the formula (I).

**6.** The composition as claimed in one or more of claims 1 to 5, which additionally comprises one or more sorbitan esters of sorbitan and carboxylic acids $R^aCOOH$, where $R^a$ is a straight-chain or branched saturated alkyl group having 5 to 11 carbon atoms or a straight-chain or branched mono- or polyunsaturated alkenyl group having 5 to 11 carbon atoms, and the weight ratio of the one or more compounds of the formula (I) to the one or more sorbitan esters just mentioned is from 70:30 to 100:0.

**7.** The composition as claimed in claim 6, wherein the one or more sorbitan esters of sorbitan and carboxylic acids $R^aCOOH$ are selected from sorbitan esters of sorbitan and caprylic acid.

**8.** The composition as claimed in one or more of claims 1 to 7, which comprises the one or more compounds of component a) in amounts of from 10.0 to 90.0% by weight and the one or more substances of component b) in amounts of from 10.0 to 90.0% by weight, in each case based on the total weight of the composition.

**9.** The composition as claimed in one or more of claims 1 to 8, wherein it is a cosmetic, dermatological or pharmaceutical composition, a crop protection formulation or a washing and cleaning composition.

**10.** The composition as claimed in claim 9, which comprises the one or more compounds of component a) in amounts of from 0.01 to 10.0% by weight and the one or more substances of component b) in amounts of from 0.01 to 10.0% by weight, in each case based on the total weight of the composition.

**11.** The composition as claimed in claim 9 or 10, wherein it is formulated on an aqueous or aqueous- alcoholic basis or is present as a solution, emulsion or dispersion and is preferably present as an emulsion.

**12.** The composition as claimed in one or more of claims 9 to 11, wherein it has a pH of from 2 to 11.

**13.** The use of a composition as claimed in claim 8 for preserving a cosmetic, dermatological or pharmaceutical composition, a crop protection formulation or a washing or cleaning composition.

**14.** The use as claimed in claim 13, wherein the cosmetic, dermatological or pharmaceutical composition, the crop protection formulation or the washing or cleaning composition is preserved against bacteria, yeasts and fungi and preferably against yeasts and fungi.

## Revendications

**1.** Composition contenant

a) un ou plusieurs composés de formule (I)

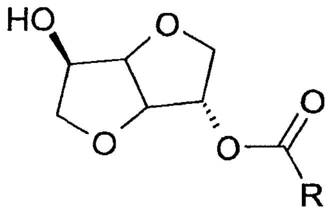

(I)

le radical R dans la formule (I) représentant un radical alkyle linéaire saturé comprenant 7 à 9 atomes de carbone et

b) un ou plusieurs diols vicinaux aliphatiques, choisis dans le groupe constitué par le 1,2-décanediol, l'éthylhexylglycérol, le 1,2-octanediol, le 1,2-hexanediol, le caprylate de glycéryle et le caproate de glycéryle,

et la composition contenant, outre ledit un ou lesdits plusieurs composés de formule (I), un ou plusieurs composés choisis dans le groupe constitué par le sorbitol, les esters de sorbitol, le sorbitane, les esters de sorbitane, l'isosorbide, les diesters d'isosorbide et les acides carboxyliques, les acides carboxyliques eux-mêmes ainsi que les acides carboxyliques à la base du composant acide des esters mentionnés correspondant à la formule RCOOH, dans laquelle R présente la signification indiquée pour la formule (I) et l'indice d'OH du mélange dudit un ou desdits plusieurs composés de formule (I) et dudit un ou desdits plusieurs composés choisis dans le groupe constitué par le sorbitol, les esters de sorbitol, le sorbitane, les esters de sorbitane, l'isosorbide, les diesters d'isosorbide et les acides carboxyliques étant inférieur ou égal à 245.

**2.** Composition selon la revendication 1, **caractérisée en ce que** le radical R dans la formule (I) représente un radical alkyle linéaire saturé comprenant 7 atomes de carbone.

**3.** Composition selon la revendication 1, **caractérisée en ce que** ladite une ou lesdites plusieurs substances du composant b) sont choisies dans le groupe constitué par le 1,2-décanediol, l'éthylhexylglycérol, le 1,2-octanediol et le 1,2-hexanediol.

**4.** Composition selon la revendication 3, **caractérisée en ce que** la substance du composant b) est l'éthylhexylglycérol.

**5.** Composition selon l'une ou plusieurs des revendications 1 à 4, **caractérisée en ce qu'**elle contient

I) 0,05 à 0,7 partie en poids d'isosorbide et

II) 0,1 à 1,0 partie en poids de diester d'isosorbide de formule

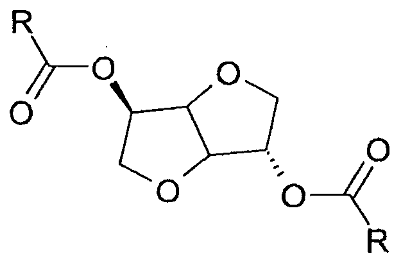

dans laquelle R a la signification indiquée pour la formule (I),

à chaque fois par rapport à 1,0 partie en poids dudit un ou desdits plusieurs composés de formule (I).

**6.** Composition selon l'une ou plusieurs des revendications 1 à 5, **caractérisée en ce qu'**elle contient en outre un ou plusieurs esters de sorbitane constitués par du sorbitane et des acides carboxyliques $R^aCOOH$, $R^a$ représentant un groupe alkyle linéaire ou ramifié, saturé, comprenant 5 à 11 atomes de carbone ou un groupe alcényle linéaire

ou ramifié, mono-insaturé ou polyinsaturé, comprenant 5 à 11 atomes de carbone et le rapport pondéral dudit un ou desdits plusieurs composés de formule (I) audit un ou auxdits plusieurs esters de sorbitane susmentionnés étant de 70:30 à 100:0.

7. Composition selon la revendication 6, **caractérisée en ce que** ledit un ou lesdits plusieurs esters de sorbitane constitués par du sorbitane et des acides carboxyliques $R^aCOOH$ sont choisis parmi les esters de sorbitane constitués par du sorbitane et de l'acide caprylique.

8. Composition selon l'une ou plusieurs des revendications 1 à 7, **caractérisée en ce qu'**elle contient ledit un ou lesdits plusieurs composés du composant a) en des quantités de 10,0 à 90,0% en poids et ladite une ou lesdites plusieurs substances du composant b) en des quantités de 10,0 à 90,0% en poids, à chaque fois par rapport au poids total de la composition.

9. Composition selon l'une ou plusieurs des revendications 1 à 8, **caractérisée en ce qu'**il s'agit d'une composition cosmétique, dermatologique ou pharmaceutique, d'une formulation de phytoprotection ou d'un agent de lavage et de nettoyage.

10. Composition selon la revendication 9, **caractérisée en ce qu'**elle contient ledit un ou lesdits plusieurs composés du composant a) en des quantités de 0,01 à 10,0% en poids et ladite une ou lesdites plusieurs substances du composant b) en des quantités de 0, ou à 10,0% en poids, à chaque fois par rapport au poids total de la composition.

11. Composition selon la revendication 9 ou 10, **caractérisée en ce qu'**elle est à base aqueuse ou aqueuse-alcoolique ou qu'elle se trouve sous forme de solution, d'émulsion ou de dispersion et de préférence sous forme d'émulsion.

12. Composition selon l'une ou plusieurs des revendications 9 à 11, **caractérisée en ce qu'**elle présente une valeur de pH de 2 à 11.

13. Utilisation d'une composition selon la revendication 8 pour la conservation d'une composition cosmétique, dermatologique ou pharmaceutique, d'une formulation de phytoprotection, d'un agent de lavage ou de nettoyage.

14. Utilisation selon la revendication 13, **caractérisée en ce que** la composition cosmétique, dermatologique ou pharmaceutique, la formulation de phytoprotection ou l'agent de lavage ou de nettoyage est conservé(e) par rapport aux bactéries, aux levures et aux champignons et de préférence-par rapport aux levures et aux champignons.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2010108738 A2
- DE 102009022444
- DE 102009022445
- JP 8173787 A
- JP 8187070 A


### In der Beschreibung aufgeführte Nicht-Patentliteratur


- **F.C. KULL et al.** *Applied Microbiology,* 1961, vol. 9, 538